(19) **Europäisches Patentamt / European Patent Office / Office européen des brevets**

(11) **EP 3 889 601 A1**

(12) **EUROPEAN PATENT APPLICATION**
published in accordance with Art. 153(4) EPC

(43) Date of publication:
06.10.2021 Bulletin 2021/40

(51) Int Cl.:
*G01N 33/49* (2006.01)   *G01N 33/53* (2006.01)

(21) Application number: 19891226.3

(86) International application number:
PCT/JP2019/046410

(22) Date of filing: 27.11.2019

(87) International publication number:
WO 2020/111135 (04.06.2020 Gazette 2020/23)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.11.2018 JP 2018223657

(71) Applicant: SYSMEX CORPORATION
Kobe-shi
Hyogo 651-0073 (JP)

(72) Inventors:
• OKA, Yuma
Kobe-shi, Hyogo 651-0073 (JP)
• YANAGIDA, Masatoshi
Kobe-shi, Hyogo 651-0073 (JP)
• MIWA, Keiko
Kobe-shi, Hyogo 651-0073 (JP)

(74) Representative: De Clercq & Partners
Edgard Gevaertdreef 10a
9830 Sint-Martens-Latem (BE)

(54) **METHOD FOR ANALYZING IMMUNE CELLS AND CELL ANALYSIS INSTRUMENT**

(57)    The present invention relates to a method for analyzing immune cells, comprising the steps of mixing whole blood containing immune cells with an immunostimulatory factor *in vitro* and labeling target molecules of the immune cells, and acquiring information on localization of the target molecules on the immune cells by detecting the label. The present invention relates to an instrument that analyses cells obtained by mixing whole blood containing immune cells with an immunostimulatory factor *in vitro* to localize the target molecules on the immune cells.

Fig. 1

| | Immunostimulatory factor and trapping body are shared | Immunostimulatory factor and trapping body are separated |
|---|---|---|
| Direct bond of trapping body to labeling substance | **A** Immunostimulatory factor and trapping body; Immune cell; Labeling substance; Target molecule | **C** Immunostimulatory factor; Labeling substance; Trapping body; Target molecule |
| Indirect bond of trapping body to labeling substance | **B** Immunostimulatory factor and trapping body; Labeling substance; Substance that specifically binds to trapping body; Target molecule | **D** Immunostimulatory factor; Trapping body; Labeling substance; Substance that specifically binds to trapping body; Target molecule |

EP 3 889 601 A1

**Description**

TECHNICAL FIELD

The present invention relates to a method for analyzing immune cells and a cell analysis instrument.

BACKGROUND ART

Immune cells are cells that play a central role in the immune system of the living body. Immune cells are also associated with various diseases such as cancer and autoimmune diseases, and in these diseases, therapeutic methods for regulating the activity and function of immune cells are known. For example, in the field of cancer, therapeutic methods including administering an immune checkpoint inhibitor to activate T cells suppressed by cancer cells have attracted attention. In the field of autoimmune diseases, therapeutic methods including administering an immunosuppressive agent to suppress the function of immune cells that cause the diseases have been performed. In these diseases, in order to determine the treatment policy and monitor the therapeutic effect, it is becoming more important to accurately grasp the activity of immune cells from a patient, in particular the stimulus responsiveness of immune cells.

In recent years, attention has been focused on measuring the immunological synapse (IS) as one of the indicators of stimulus responsiveness of an immune cell. The IS is a structure formed on an immune cell at the contact surface when the immune cell comes into contact with a target cell. The IS-forming ability is known to correlate with the cytokine-secreting ability of an immune cell. The formation of IS occurs within minutes to tens of minutes after contact of an immune cell with a target cell, and is therefore considered to be an important process in the early stage of immune cell activation.

It is known that a structure similar to the IS is formed on an immune cell by contact of the immune cell with an immunostimulatory factor and a foreign substance. Patent Document 1 discloses that seeding a T cell clone prepared from a peripheral blood mononuclear cell (PBMC) on a plate onto which an anti-CD3 antibody has been immobilized and adding an anti-CD28 antibody to cause stimulation with the antibody and contact with the bottom of the plate result in localization of CD3 and CD28 molecules on the T cell clone. In Patent Document 1, the localization of target molecule (CD3 and CD28) caused on the T cell clones is detected as a structure corresponding to the IS. Specifically, immunostaining the T cell clone collected from the plate with a fluorescently labeled antibody that binds to CD3 and CD28, and performing measurement with a flow cytometer to evaluate the immunostimulus responsiveness of immune cells allow the localization of the CD3 and CD28 molecules on the cell to be detected.

PRIOR ART DOCUMENTS

PATENT DOCUMENTS

Patent Document 1: US 2018/0292385

DISCLOSURE OF THE INVENTION

PROBLEMS TO BE SOLVED BY THE INVENTION

The method described in Patent Document 1 includes measuring isolated immune cells, such as a T cell clone prepared from a PBMC. When analyzing the immune cells from a subject by this method, for example, the immune cells contained in blood collected from the subject are targeted for analysis. However, the breakdown of blood cell components in blood is that erythrocytes account for 90% or more, and immune cells such as leukocytes account for several%. As described above, since erythrocytes are overwhelmingly abundant as compared with immune cells in blood, blood cells other than the immune cells may influence IS formation on the immune cells. To eliminate such an influence, immune cells need to be previously separated and isolated from blood.

However, the operation of separating and isolating immune cells from a biological sample such as blood is complicated and time-consuming. In addition, the collection rate of immune cells varies depending on an operator. In addition, reagents used for isolation and isolation of immune cells and physical treatments such as centrifugation may damage the immune cells, and thus reduce the quality and function of the immune cells, making it impossible to accurately measure the activity of the immune cells. Therefore, there is a demand for a method for analyzing immune cells without requiring a step of separating and isolating the immune cells from a biological sample.

SOLUTIONS TO THE PROBLEMS

The present inventors have investigated whether stimulation of immune cells in whole blood by adding an immunostimulatory factor to the whole blood collected from a subject influences localization of target molecule. As a result, surprisingly, the present inventors have found that even when immune cells are stimulated in whole blood, target molecules localize on the immune cells and this localization can be detected.

One aspect of the present invention provides a method for analyzing immune cells, including the steps of mixing whole blood containing immune cells with an immunostimulatory factor *in vitro* and labeling target molecules of the immune cells, and acquiring information on localization of target molecule on the immune cells by detecting a label.

One aspect of the present invention provides a method for analyzing an immune cell, including the step of acquiring information on localization of target molecule on the immune cell by detecting a label of the immune cell comprising a labeled target molecule. In this method, the immune cells containing the labeled target molecules are cells prepared by mixing whole blood containing immune cells with an immunostimulatory factor *in vitro* and labeling target molecules of the immune cells contained in the whole blood.

One aspect of the present invention provides a cell analysis instrument including an introducing unit that introduces a complex of an immune cell, a trapping body that binds to a target molecule of the immune cell, and a labeling substance that can generate a detectable optical signal, an image capturing unit that captures the complex supplied from the introducing unit, and an analyzing unit that acquires information on localization of target molecule on the immune cell based on an image captured by the image capturing unit. In this instrument, the immune cells are cells obtained by mixing whole blood containing immune cells with an immunostimulatory factor *in vitro* to localize the target molecules on the immune cells.

One aspect of the present invention provides a cell analysis instrument including an introducing unit that introduces a complex of an immune cell, a trapping body that binds to a target molecule of the immune cell, and a labeling substance that can generate a detectable optical signal, a detection unit that irradiates the complex supplied from the introducing unit with light to detect the optical signal generated from the complex, and an analyzing unit that acquires information on the localization of target molecule on the immune cell based on the detected optical signal. In this instrument, the immune cells are cells obtained by mixing whole blood containing immune cells with an immunostimulatory factor *in vitro* to localize the target molecules on the immune cells.

EFFECTS OF THE INVENTION

According to the present invention, the step of separating and isolating immune cells from a biological sample becomes unnecessary, so that the time required for analysis is shortened. In addition, since damage to immune cells can be minimized, deterioration of the quality and function of immune cells can be prevented, and the state of immune cells can be analyzed more accurately.

BRIEF DESCRIPTION OF THE DRAWINGS

Fig. 1 is a schematic view illustrating an example of labeling of a target molecule on an immune cell to be analyzed.
Fig. 2 is a view showing a configuration of a cell analysis instrument.
Fig. 3 is a diagram showing a configuration of a measuring device.
Fig. 4 is a view showing a configuration of an image capturing unit.
Fig. 5 is a schematic view showing a region on the light receiving surface of a camera.
Fig. 6 is a diagram showing a configuration of an information processing device.
Fig. 7 is a flowchart showing measurement processing and analysis processing of a sample.
Fig. 8 is an example of image showing measurement data.
Fig. 9 is a schematic view showing a measurement process based on measurement data.
Fig. 10 is a flowchart showing analysis processing.
Fig. 11 is a 2D scattergram showing analysis processing according to Embodiment 1 (a, b and c) and Embodiment 2 (d).
Fig. 12 is a view showing a configuration of a detection unit as a modified example of the image capturing unit.
Fig. 13 is a schematic view for explaining pulse data of fluorescence signals obtained from cells having different distribution states of co-stimulatory molecules.
Fig. 14 is schematic diagrams for explaining 2D scattergrams of parameters for analysis obtained from pulse data of fluorescence signals.
Fig. 15A is a 2D scattergram showing the distribution of immune cells (T cells) in whole blood to which an immunostimulatory factor (anti-CD28 antibody) has not been added.

Fig. 15B is a 2D scattergram showing the distribution of T cells in whole blood to which an anti-CD28 antibody has been added.

Fig. 16A is an example of images showing the expression distribution of CD28 and CD3 on T cells in whole blood to which an anti-CD28 antibody has not been added.

Fig. 16B is an example of images showing the expression distribution of CD28 and CD3 on T cells in whole blood to which an anti-CD28 antibody has been added.

Fig. 17 is a graph showing the ratio of T cells on which CD28 localizes to T cells in whole blood (CD28 localization positive rate).

Fig. 18 is a graph showing the ratio of T cells on which CD28 localizes to T cells in whole blood or peripheral blood mononuclear cells (PBMCs) isolated from whole blood (CD28 localization positive rate).

Fig. 19 is a graph showing the ratio of T cells on which CD28 localizes to T cells in whole blood or PBMCs isolated from whole blood to which an anti-CD28 antibody has not been added (CD28 localization positive rate).

Fig. 20A is a graph showing the ratio of a T cell clone on which CD28 localizes to a T cell clone added to whole blood or a plate (CD28 localization positive rate).

Fig. 20B is a graph showing the ratio of a T cell clone on which CD28 localizes to a T cell clone added to whole blood or a plate (CD28 localization positive rate).

Fig. 21 is a graph showing the ratio of a T cell clone on which CD28 localizes to a T cell clone added to whole blood or a plate (CD28 localization positive rate).

Fig. 22 is a graph showing the ratio of a T cell clone on which CD28 localizes to a T cell clone added to whole blood or a plate (CD28 localization positive rate).

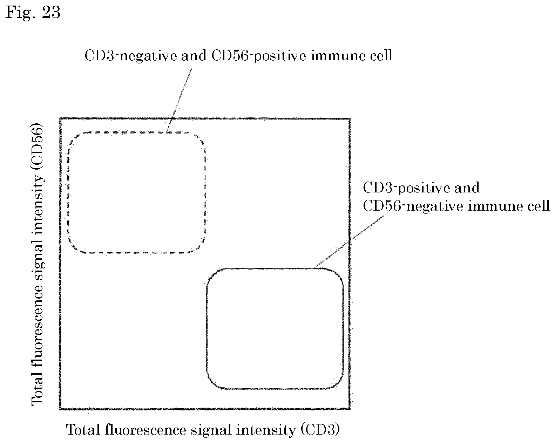

Fig. 23 is a schematic diagram showing in a 2D scattergram a region in which dots representing CD3-negative and CD56-positive immune cells appear, and a region in which dots representing CD3-positive and CD56-negative immune cells appear.

Fig. 24 is 2D scattergrams showing the distribution of T cells and NK cells in whole blood to which human B cells have been added or have not been added.

Fig. 25 is an example of images showing the expression distribution of CD3 and F-actin on a T cell in whole blood and the expression distribution of CD56 and F-actin on an NK cell in whole blood.

Fig. 26A is a graph showing the ratio of T cells on which F-actin localizes to T cells in whole blood (F-actin localization positive rate).

Fig. 26B is a graph showing the ratio of NK cells on which F-actin localizes to NK cells in whole blood (F-actin localization positive rate).

MODE FOR CARRYING OUT THE INVENTION

[1. Method for analyzing immune cells]

(Step of causing localization of target molecule on immune cells)

In a method for analyzing immune cells of the invention (hereinafter, referred to as "method"), immune cells obtained through the following steps are analyzed: a step of mixing whole blood containing immune cells with an immunostimulatory factor *in vitro,* and a step of labeling target molecules of the immune cells. Hereinafter, a description is made of this step of labeling the target molecule.

The whole blood may be blood collected from human or a non-human mammal. Examples of the whole blood include peripheral blood and umbilical cord blood. Peripheral blood is preferable. As needed, an anticoagulant and/or a chelating agent may be added to whole blood. Examples of the anticoagulant include heparin, citric acid and citrate. Examples of the chelating agent include an EDTA (ethylene diamine tetraacetic acid) salt.

Immune cells mean cells responsible for the immune system, which is a mechanism that performs self-identification and non-self-identification, and eliminates non-self. The immune cells to be analyzed in the method of the invention (hereinafter, also referred to as "immune cells to be analyzed") can be freely selected from publicly known immune cells. The immune cells to be analyzed may be one cell, but are preferably a subpopulation (subset) substantially composed of a plurality of the same kind of immune cells. In the art, immune cells are classified into various subsets according to their function and/or morphology. Examples of the subset of immune cells include subsets of lymphocytes, granulocytes, monocytes and the like. Lymphocytes are further classified into subsets of T cells, natural killer (NK) cells and B cells. Granulocytes are further classified into subsets of neutrophils, eosinophils and basophils.

The immune cells to be analyzed may be immune cells originally contained in whole blood collected from a subject, or may be immune cells added from the outside to whole blood. The immune cells to be analyzed are preferably immune cells involved in cell-mediated immunity, and examples thereof include T cells and NK cells. Among them, T cells are particularly preferable.

The T cells include effector T cells such as helper T cells (also referred to as CD4$^+$ T cells), regulatory T cells (also referred to as T$_{reg}$ cells) and cytotoxic T cells (also referred to as killer T cells or CD8$^+$ T cells), naive T cells, and genetically modified T cells. Effector T cells may be T cells activated either *in vivo* or *in vitro.* The T cells may be of one kind, or two or more kinds.

As used herein, the term "immunostimulatory factor" refers to a substance that can act on immune cells to induce activation of the immune cells. The activation of immune cells refers to, for example, proliferation of immune cells, and enhancement of motility and cytokine secretion. Examples of the immunostimulatory factors include immunostimulatory antibodies, immunostimulatory peptides and major histocompatibility molecules (hereinafter, also referred to as "MHC molecules"). In addition, immunostimulatory factors present on allogeneic cells, antigen-presenting cells and tumor cells may be used. The immunostimulatory factor may be one kind or a combination of two or more kinds. The immunostimulatory factor may be used in a form bound to or combined with a substance different from that of immune cells. Examples of the form include a form of a complex of an MHC molecule with an antigenic peptide, a form immobilized on a non-biological material such as beads or a container, and a form presented on an antigen-presenting cell.

As used herein, the term "immunostimulatory antibody" refers to an antibody that can specifically stimulate immune cells to induce activation of the immune cells. Examples of the immunostimulatory antibody include, but are not limited to, an anti-CD28 antibody, an anti-CD3 antibody, an anti-TCRα/β antibody, an anti-CD40L (CD40 ligand) antibody, an anti-OX40 antibody, an anti-CD2 antibody, an anti-CD 13 7 antibody, an anti-CTLA4 (cytolytic T lymphocyte associated antigen-4) antibody, an anti-PD-1 (programmed cell death-1) antibody, an anti-ICOS (inducible co-stimulatory molecule) antibody, and an anti-integrin antibody (anti-CD2 antibody). In particular embodiments, an anti-CD28 antibody and an anti-CD3 antibody are preferable. These immunostimulatory antibodies may be used alone or in combination of two or more. The immunostimulatory antibody may be used in a state of being immobilized on a foreign substance of a non-biological material described later.

In the present specification, the antibody may be either a monoclonal antibody or a polyclonal antibody unless otherwise specified. In addition, the antibody may be an antibody fragment such as Fab or F(ab')2, a single chain antibody, or a derivative thereof.

As used herein, the term "immunostimulatory peptide" refers to a peptide that can stimulate immune cells to induce activation of the immune cells. The peptide also includes protein. The immunostimulatory peptide is, for example, an antigenic peptide (such as an antigenic peptide of *Mycobacterium tuberculosis*). The antigenic peptide may be used alone or in combination of two or more. The antigenic peptide may be used in the form of a complex with an MHC molecule presented on the MHC molecule.

As used herein, the term "major histocompatibility molecule" (MHC molecule) refers to a molecule responsible for alloantigenicity that causes the strongest rejection in allogeneic transplantation of an organ, a tissue and cells. The HLA molecule in humans or the H-2 molecule in mice corresponds to the MHC molecule. The MHC molecule includes a class I molecule and a class II molecule. The MHC class I molecule is expressed on almost all nucleated cells, forms a complex with an intracellularly produced peptide, and presents the complex to the receptor of a CTL (CD8$^+$ T cell). The MHC class II molecule is expressed only on a so-called antigen-presenting cell such as a macrophage and a B cell, and presents a peptide derived from a foreign antigen to the receptor of a helper T cell (CD4$^+$ T cell).

In one embodiment, when the immunostimulatory factor is an MHC molecule or a complex of an MHC molecule with an antigenic peptide, the MHC molecule may be either an MHC class I molecule or an MHC class II molecule. The MHC molecule or the complex of an MHC molecule with an antigenic peptide may be used in a form existing in the cell membrane of, for example, an antigen-presenting cell or a tumor cell.

As used herein, the term "immunostimulation" includes bringing an immune cell into contact with an immunostimulatory factor. The form of bringing an immune cell into contact with an immunostimulatory factor is not particularly limited, but examples thereof include bond, association or complex formation of a molecule on an immune cell to/with an immunostimulatory factor, and uptaking an immunostimulatory factor by an immune cell. The molecule on an immune cell that comes into contact with an immunostimulatory factor may be a target molecule described later, or may be a molecule different from the target molecule.

In a preferred embodiment, the immunostimulation includes bringing the immune cell in contact with an immunostimulatory factor into contact with a foreign substance. Here, the "foreign substance" refers to a substance different from the immune cells to be analyzed. Since whole blood is usually accommodated in a container for use, mixing the whole blood with an immunostimulatory factor *in vitro* results in both contact of immune cells with the immunostimulatory factor, and contact of immune cells in contact with the immunostimulatory factor with the inner surface of the container as the foreign substance.

The substance different from the immune cells to be analyzed may be a non-biological material or a biological material. The foreign substance when non-biological material is not particularly limited, but may be, for example, a container such as a tube, a multi-well plate or a dish, a slide glass, or beads. Preferably, the foreign substance is a container for accommodating collected whole blood. The foreign substance when biological material is not particularly limited, but may be, for example, a blood cell, an allogeneic cell, an antigen-presenting cell or a tumor cell, other than an immune cell having the target molecule labeled. Examples of the blood cell other than an immune cell having the target molecule labeled includes an erythrocyte, and an immune cell not to be analyzed. For example, an NK cell, a granulocyte, a monocyte, a B cell and the like correspond to the immune cell not to be analyzed, when the immune cell having the target molecule labeled, that is, the immune cell to be analyzed is a T cell. The allogeneic cell is a non-self cell, and may be a cell derived from an individual different from an individual from which the whole blood used in the method of the invention has been collected. The blood cell, allogeneic cell, antigen-presenting cell and tumor cell other than the immune cell to be analyzed may be an established cell. The allogeneic cells and tumor cells may be in the form of a tissue containing the same.

In particular embodiments, mixing whole blood containing immune cells with an immunostimulatory factor *in vitro* immunostimulates an immune cell to cause localization of target molecule on the immune cells. That is, the step of labeling target molecules includes a step of mixing whole blood containing immune cells with an immunostimulatory factor *in vitro* to cause localization of target molecule on the immune cells. The method for mixing whole blood with an immunostimulatory factor *in vitro* is not particularly limited, as long as immune cells in the whole blood and the immunostimulatory factor can come into contact with each other. For example, an immunostimulatory factor may be added to collected whole blood. When an immunostimulatory factor is present on a substance different from immune cells, whole blood containing the immune cells may be brought into contact with the substance *in vitro*. In this case, the immune cells in the whole blood come into contact with the immunostimulatory factor and at the same time come into contact with the substance different from the immune cells.

The amount of the immunostimulatory factor can be appropriately determined depending on the type of the immunostimulatory factor. In particular embodiments, the amount is not particularly limited as long as immune cells can be sufficiently activated.

In the method of the invention, the stimulation time with an immunostimulatory factor is not particularly limited, but the upper limit is, for example, less than 6 hours, preferably 3 hours or less, more preferably 2 hours or less. The lower limit of the stimulation time is, for example, 2 minutes or more, preferably 5 minutes or more, more preferably 10 minutes or more. Here, the stimulation time is, for example, a time from mixing whole blood containing immune cells with an immunostimulatory factor *in vitro* to labeling target molecules of the immune cells. In a preferred embodiment, the stimulation time is a time from mixing whole blood containing immune cells with an immunostimulatory factor *in vitro* to fixation of the immune cells.

Immunostimulating immune cells contained in whole blood promotes localization of target molecule on the immune cells. As used herein, the term "target molecule" refers to a molecule expressed on an immune cell, in which the distribution of the molecule on the immune cell can be changed by immunostimulation. The term "localization of target molecule" refers to a group of target molecule formed by gathering of the target molecule on a part of an immunostimulated immune cell. As the site where the target molecules localize on an immune cell, a part of the cell membrane of the immune cell is preferable. Examples of the target molecules include molecules constituting an immunological synapse. Examples of the molecules constituting an immunological synapse include a surface antigen and a polymer of structural protein. The method of the invention can evaluate the immunostimulus responsiveness of immune cells using the localization of target molecule caused by immunostimulation as an index.

The immunological synapse refers to a molecular complex that is formed on the surface of an immune cell and plays a role in activation. The immunological synapse is known to be formed by gathering of a receptor (e.g., a T cell receptor (TCR) or an NK cell receptor), a cofactor of the receptor (e.g., CD3), various co-stimulatory molecules (e.g., CD28), a structural protein (e.g., actin), an adhesion molecule (e.g., integrin) and a signaling molecule on a part of the cell surface. IS can be formed on immune cells by bringing cells targeted by the immune cells into contact with the immune cells. In addition, not limited to this, IS can also be formed by bringing into contact with a molecule that induces IS formation (e.g., an immunostimulatory factor), a non-biological material, or the like. As used herein, the term "localization of target molecule" and "immunological synapse" are synonymous. Even after the immune cells are not in contact with the other cell or non-biological material but become independent, IS remains on the cells for a while (about 30 minutes).

The surface antigen is a protein that has an extracellular domain. The surface antigen may have an intracellular domain, a trans-membrane domain, and the like. The surface antigen can be a protein, a sugar chain, a lipid and a combination thereof that are present on the cell membrane. The type of surface antigen is not particularly limited as long as a trapping body capable of binding to the surface antigen exists or such a trapping body can be produced. A preferred surface antigen is molecules constituting an immunological synapse on an immune cell. Examples of the surface antigen include CD3, CD28, TCRα/β, CD40L, OX40, CTLA4, PD-1 and ICOS. The surface antigen may be a co-stimulatory molecule. The co-stimulatory molecule is a type of surface antigen of immune cells. For example, a T cell co-stimulatory molecule is a molecule that can give a T cell an auxiliary signal to activate the T cell, in addition to an antigen-specific signal mediated by TCR. An NK cell co-stimulatory molecule is a molecule that can give an NK cell an auxiliary signal to activate the NK cell, in addition to a target cell ligand-specific signal mediated by an activated receptor of an NK cell.

Examples of the co-stimulatory molecule include CD28, OX40, CD40L, CTLA4, PD-1 and ICOS.

The structural protein refers to a protein involved in maintenance and regulation of the structure or form of cells, an organ or the like in the living body. Examples of the structural protein include actin and tubulin. In particular embodiments, as the polymer of the structural protein, a polymer of a protein constituting the cytoskeleton is preferable, and F-actin is particularly preferable. F-actin localizes on a part of the cell membrane on the side of cytoplasm in immunostimulated immune cells.

The localization of target molecule on immune cells is facilitated by bringing immune cells in contact with an immunostimulatory factor into contact with a foreign substance. In particular embodiments, it is preferable to perform a step of mixing whole blood with an immunostimulatory factor *in vitro,* and then bringing immune cells into contact with a substance different from the immune cells. This step can be performed, for example, by centrifuging, stirring, or allowing to stand a mixture of the whole blood containing immune cells, and the immunostimulatory factor. As a result, the immune cells to be analyzed can come into contact with blood cells other than the immune cells to be analyzed, and the inner surface of a container accommodating the whole blood. The contact may be made within a range of, for example, 2 minutes or more and 120 minutes or less.

The stirring of whole blood may be any of inversion mixing, pipetting, stirring with a stirrer, and the like. Inversion mixing is preferable. The inversion mixing may be performed by a hand method or by using a rotator.

The rotational speed of centrifugation or centrifugal acceleration is not particularly limited, but mild conditions that do not damage immune cells are preferable. For example, centrifugation may be performed under a condition in which whole blood is separated into a plasma component and a cell component. In this case, the cell component is separated into a layer containing immune cells such as leukocytes (buffy coat) and a layer containing erythrocytes. As a result, the immune cells to be analyzed in the buffy coat can efficiently come into contact with other leukocytes. The other erythrocytes are immune cells that are contained in the buffy coat and not to be analyzed. After the contact, the separated whole blood may be stirred.

In particular embodiments, mixing whole blood containing immune cells with an immunostimulatory factor *in vitro* causes localization of target molecule on cells that are responsive to the immunostimulation among the immune cells. For example, the CD28 molecules are usually uniformly distributed on the cell membrane of non-immunostimulated T cells. On the other hand, when T cells are immunostimulated by coming into contact with an anti-CD28 antibody as the immunostimulatory factor and a foreign substance, the CD28 molecules tend to localize at a position in contact with the anti-CD28 antibody on the cell membrane.

In particular embodiments, it is preferable to fix immune cells contained in whole blood between the step of labeling target molecules of the immune cells and the step of acquiring information on the localization of target molecule on the immune cells described later. The caused localization of target molecule on the immune cells continues for a while, and the caused localization of target molecule can be more reliably maintained by performing a fixing treatment.

Fixation of immune cells can be performed by adding a fixing agent to whole blood mixed with an immunostimulatory factor. The fixing agent may be any reagent usually used for immunostaining cells or a tissue, and examples thereof include paraformaldehyde and lower alcohols. A commercially available cell fixing solution may be used.

In particular embodiments, it is preferable to dissolve erythrocytes contained in whole blood between the step of labeling target molecules of immune cells and the step of acquiring information on the localization of target molecule on the immune cells described later. As mentioned above, since erythrocytes are much more abundant in whole blood than immune cells, immune cells can be more accurately measured by lysing erythrocytes. In a preferred embodiment, in order to reduce the effect on the caused localization of target molecule on immune cells, erythrocytes contained in whole blood are lysed between the step of fixing immune cells contained in whole blood and the step of acquiring information on the localization of target molecule on the immune cells described later.

Lysis of erythrocyte can be performed by adding a hemolytic agent to whole blood mixed with an immunostimulatory factor. The hemolytic agent may be any reagent that destroys the cell membrane of erythrocytes for hemolysis, but does not substantially damage immune cells. Examples of such a hemolytic agent include ammonium chloride, surfactants and mixtures thereof. As the surfactants, a cationic surfactant, a nonionic surfactant, or a combination thereof is preferable. Reagents containing a hemolytic agent are commercially available. For example, a reagent for measuring blood cells publicly known in the field of blood testing, such as a reagent for classifying leukocytes may be used.

In particular embodiments, as needed, permeabilization treatment of the cell membrane of immune cells may be performed. This allows a trapping body and a labeling substance to enter immune cells and label target molecules inside the cells. This also enables labeling of cell nuclei. The permeabilization treatment of the cell membrane itself is publicly known, and examples thereof include a method using a surfactant such as saponin, Triton(trademark) X-100 or Tween(trademark) 20. When a hemolytic agent containing a surfactant is used, lysis of erythrocytes and permeabilization treatment of the cell membrane of immune cells can be performed at the same time.

In the method of the invention, it is preferable to mix whole blood containing immune cells with an immunostimulatory factor *in vitro* and then label target molecules of the immune cells.

In a preferred embodiment, target molecules are labeled by binding the target molecules of immune cells, a trapping body that binds to the target molecule, and a labeling substance that can generate a detectable optical signal. In this case, the labeling substance indirectly binds to the target molecule via the trapping body, whereby the target molecule is labeled.

A specific operation of labeling target molecules may include adding a trapping body that binds to the target molecule and a labeling substance that can generate a detectable optical signal to whole blood containing immune cells that have been immunostimulated by an immunostimulatory factor. That is, the method of the invention is advantageous in that it is not necessary to isolate immune cells from whole blood after immunostimulation for labeling of target molecule, and the number of steps and time required for operation can be reduced.

The trapping body that binds to a target molecule (hereinafter, also simply referred to as "trapping body") can be appropriately determined according to the type of the target molecule. A preferred trapping body is substances that can specifically bind to a target molecule, and examples thereof include antibodies, aptamers, receptor molecules and ligand molecules. When the target molecule is F-actin, c may be used as the trapping body. Phalloidin is a peptide composed of 7 amino acids that specifically binds to F -actin.

As the labeling substance that can generate a detectable optical signal (hereinafter, also simply referred to as "labeling substance"), a fluorescent substance is preferable. The fluorescent substance can be appropriately selected from publicly known fluorescent dyes such as phycoerythrin (PE), allophycocyanin (APC), fluorescein isothiocyanate (FITC), rhodamine, Alexa Fluor(registered trademark) and cyanine-based dyes, known fluorescent proteins such as green fluorescent protein (GFP), and the like.

The labeling substance preferably binds directly or indirectly to a trapping body. Examples of the direct bond of a labeling substance to a trapping body include labeling of a trapping body with a labeling substance. The method for labeling a substance itself is publicly known in the art. For example, a molecule in which a trapping body is covalently bonded to a labeling substance (labeled trapping body) may be prepared using a cross-linking agent or the like. When the trapping body is a protein such as an antibody, it may be labeled using a commercially available fluorescent labeling kit or the like. Alternatively, when a labeled antibody against a target molecule is commercially available, it may be used. A trapping body to which a labeling substance is directly bound binds to a target molecule, whereby the target molecule is labeled.

Examples of the indirect bond of a labeling substance to a trapping body include bond of a labeling substance to a trapping body via a substance that can specifically bind to the trapping body. In this case, the substance that can specifically bind to a trapping body is preferably labeled with a labeling substance. Examples of the substance that can specifically bind to a trapping body include antibodies and aptamers. When the trapping body is an antibody, it is preferable to use a labeled antibody that specifically binds to the trapping body. In this case, the trapping body binds to a target molecule as a primary antibody, and the labeled antibody that specifically binds to the trapping body binds as a secondary antibody to the trapping body bound to the target molecule. As a result, the labeling substance indirectly binds to the trapping body, whereby the target molecule is labeled.

Alternatively, a biotin-modified trapping body and avidins to which a labeling substance is immobilized may be used. In this case, the labeling substance can indirectly bind to the trapping body bound to a target molecule via the specific binding of biotin to avidins. The avidins refer to avidin, streptavidin and their derivatives.

In particular embodiments, as needed, the cell nuclei of immune cells may be labeled. When the cell membrane of immune cells has undergone permeabilization treatment, the cell nuclei can be stained with a fluorescent dye that can stain nucleic acid. Examples of such a fluorescent dye include Hoechst33342, Hoechst33258 and 4',6-diamidino-2-phenylindole dihydrochloride (DAPI).

In particular embodiments, the immunostimulatory factor described above may be shared as a trapping body that binds to a target molecule. As used herein, the term the immunostimulatory factor is "shared as a trapping body" means that the immunostimulatory factor also functions as a trapping body that binds to a target molecule to be used for both stimulation of immune cells and trap of the target molecule. As such an immunostimulatory factor, an immunostimulatory antibody is preferable. Using the immunostimulatory factor shared as a trapping body can localize target molecules on immune cells and trap the target molecules at the same time. In addition, when the immunostimulatory factor shared as a trapping body is labeled with a labeling substance, it is possible to localize target molecules on immune cells, capture the target molecules, and label the target molecules at the same time.

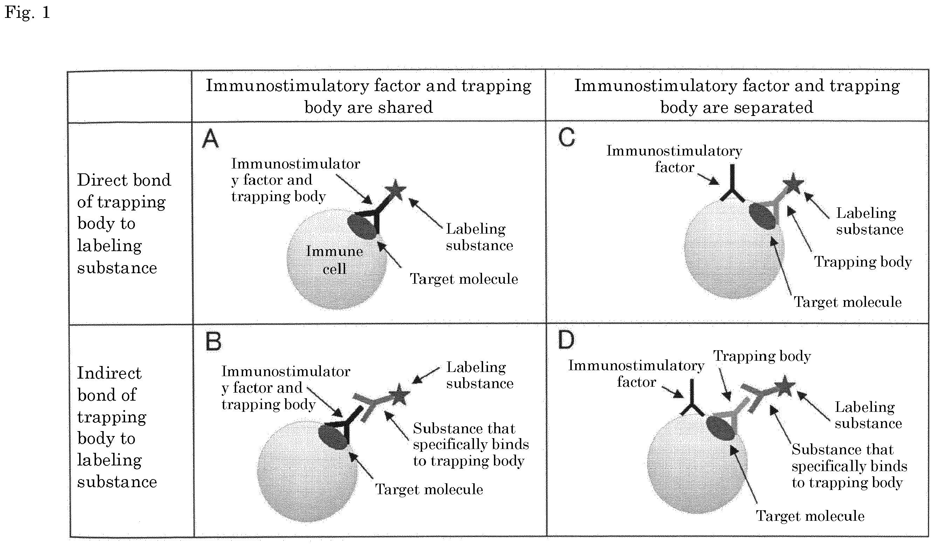

A description is made of examples of trapping and labeling target molecules in the method of the invention with reference to Fig. 1. In Fig. 1, an immunostimulatory factor, a trapping body, and a substance that specifically binds to the trapping body are all shown as antibodies, but the present invention is not limited thereto. Panels A and B in Fig. 1 represent the case where the immunostimulatory factor is shared as the trapping body. In panel A, the labeling substance directly binds to the immunostimulatory factor (trapping body). In this case, the contact of the immunostimulatory factor with an immune cell localizes a target molecule, and trap the target molecule by the immunostimulatory factor at the same time. Furthermore, the labeling substance is directly bound to the immunostimulatory factor, whereby the target molecule is labeled. In panel B, the labeling substance indirectly binds to the immunostimulatory factor (trapping body). In this case, the contact with the immunostimulatory factor localizes a target molecule, and trap the target molecule by the immunostimulatory factor at the same time. Then, the target molecule is labeled by binding as a secondary antibody a substance that specifically binds to the trapping body and that is labeled with the labeling substance, to the trapping body.

Panels C and D represent the case where the immunostimulatory factor and the trapping body are separate molecules. In panels C and D, the case where a molecule on an immune cell in contact with the immunostimulatory factor is a molecule different from the target molecule is exemplified, but the present invention is not limited to this example. In panel C, the labeling substance directly binds to the trapping body. In this case, first, the contact of the immunostimulatory factor with an immune cell localizes the target molecule. Then, the trapping body binds to the localized target molecule. Here, the labeling substance is directly bound to the trapping body, whereby the target molecule is labeled as soon as the target molecule is trapped. In panel D, the labeling substance indirectly binds to the trapping body. In this case, first, the contact of the immunostimulatory factor with an immune cell localizes the target molecule. Then, the trapping body binds to the localized target molecule. Furthermore, the target molecule is labeled by binding as a secondary antibody a substance that specifically binds to the trapping body and that is labeled with the labeling substance, to the trapping body.

(Step of acquiring information on localization of target molecule)

In the analyzing method of the invention, the immune cells obtained by the step of labeling the target molecules described above are analyzed. Specifically, information on the localization of target molecule on immune cells is acquired by detecting the label.

In particular embodiments, it is preferable to detect an optical signal generated from the labeling substance and acquire information on the localization of target molecule on immune cells based on the detected optical signal. A means for detecting the optical signal is not particularly limited, but a means capable of analyzing individual cells is preferable. Examples of such a means for detecting an optical signal include a flow cytometer (FCM) and an imaging flow cytometer (IFC). In a preferred embodiment, FCM or IFC is used to detect the optical signal.

When detecting an optical signal by FCM or IFC, whole blood containing an immunostimulatory factor, a trapping body and a labeling substance can be used as it is as a sample for measurement. That is, the method of the invention using FCM or IFC is advantageous in that it is not necessary for analysis of labeled immune cells to isolate immune cells from whole blood after immunostimulation and labeling treatment, and the number of steps and time required for operation can be reduced.

FCM is a device that can measure the size of individual cells in a cell population, the distribution of protein expression level, and the like by irradiating cells flowing in a liquid with light, and detecting scattered light signals and/or fluorescence signals emitted from the individual cells. IFC is a flow cytometer equipped with an image capturing unit such as a CCD camera, which flow cytometer is a device capable of acquiring images of cells flowing in a liquid. More specifically, IFC can acquire a fluorescence signal, a scattered light signal, a fluorescence image and a transmitted light image from each of thousands to millions of cells in a short time of seconds to minutes to perform quantitative measurement. Information on each cell can be extracted by image processing.

In particular embodiments, FCM and IFC are not particularly limited, and commercially available FCM or IFC may be used. In addition, the light source of FCM and IFC is not particularly limited, but a light source having a wavelength suitable for exciting a fluorescent substance can be appropriately selected. As the light source, for example, a blue semiconductor laser, a red semiconductor laser, an argon laser, a He-Ne laser or a mercury arc lamp is used.

In particular embodiments, by IFC, it is preferable to detect an optical signal generated from the labeling substance and acquire information on the localization of target molecule on immune cells based on the detected optical signal. As will be described later, according to IFC, multiple parameters including the fluorescence signal area value, total fluorescence signal intensity, cell size, aspect ratio and the like can be obtained based on the fluorescent and transmitted light images of the image-captured cells.

In particular embodiments, as information on the localization of target molecule on immune cells, for example, the number of immune cells on which the target molecules localize may be acquired. The number of immune cells on which the target molecules localize can be counted, for example, based on the values acquired based on the detected optical signal, which values reflect the distribution of labeled target molecule on immune cells. When the labeling substance is a fluorescent substance, examples of the values that reflect the distribution of labeled target molecule include the pulse width, pulse height and pulse area of the fluorescence signal from the labeling substance indirectly bound to the target molecule.

For example, when the target molecule is CD28, in non-immunostimulated immune cells, CD28 is usually uniformly distributed on the cell membrane, so that the pulse width is large and the pulse height is low. On the other hand, in immunostimulated immune cells, when CD28 localizes at a predetermined position on the cell membrane, the pulse width is smaller and the pulse height is higher than those of non-immunostimulated immune cells.

When the labeling substance is a fluorescent substance and IFC is used to detect the fluorescence signal, immune cells on which the target molecules localize can be counted based on fluorescence images of individual cells acquired based on fluorescence signals. For example, as the value reflecting the distribution of labeled target molecule, a fluorescence signal area value may be acquired based on the fluorescence images of individual cells. Here, the "fluorescence signal area value" is the number of pixels constituting an area showing the fluorescence signal in the fluorescence images of individual cells. Each pixel constituting an area showing the fluorescence signal has a pixel value corresponding to the fluorescence signal. In the acquisition of the fluorescence signal area value from the fluorescence image of a cell, the number of pixels having a pixel value higher than a predetermined pixel value (for example, background) may be acquired.

For example, when the target molecule is CD28, in non-immunostimulated immune cells, CD28 is usually uniformly distributed on the cell membrane, so that the fluorescence signal area value is high. On the other hand, in immunostimulated immune cells, when CD28 localizes at a predetermined position on the cell membrane, the fluorescence signal area value is lower than that of non-immunostimulated immune cells.

In particular embodiments, when the fluorescence signal area value of one immune cell is lower than a predetermined threshold value, it can be determined that the immune cell is an immune cell on which the target molecules localize. In addition, when the fluorescence signal area value of one immune cell is higher than a predetermined threshold value, it can be determined that the immune cell is an immune cell on which the target molecule does not localize. Accordingly, immune cells on which the target molecules localize can be counted by extracting data on an immune cell group whose fluorescence signal area value is lower than a predetermined threshold value from data on an immune cell group measured by IFC. When the fluorescence signal area value of one immune cell is the same as a predetermined threshold value, the immune cell may be determined to be an immune cell on which the target molecules localize, or may be determined to be an immune cell on which the target molecule does not localize.

The predetermined threshold value of the fluorescence signal area value is not particularly limited but can be appropriately set. For example, the predetermined threshold value may be empirically set by measuring a sample prepared from whole blood to which an immunostimulatory factor has been added and whole blood to which no immunostimulatory factor has been added by IFC, and accumulating data on the fluorescence signal area value.

The ratio of immune cells on which the target molecules localize to immune cells in whole blood (hereinafter, also referred to as "localization positive rate") may be calculated by dividing the number of immune cells on which the target molecules localize by the total number of measured immune cells. In particular embodiments, as information on the localization of target molecule on immune cells, the localization positive rate may be acquired.

The localization positive rate can be said to be an index of the immunostimulus responsiveness of a predetermined immune cell subset contained in whole blood. Accordingly, in particular embodiments, the immunostimulus responsiveness of an immune cell subset contained in whole blood may be determined based on the localization positive rate. As the predetermined immune cell subset, a T cell subset or an NK cell subset is preferable. For example, when the localization positive rate of a predetermined immune cell subset contained in whole blood of a subject is lower than a predetermined threshold value, it can be determined that the immunostimulus responsiveness of the predetermined immune cell subset is low. In addition, when the localization positive rate of a predetermined immune cell subset contained in whole blood of a subject is higher than a predetermined threshold value, it can be determined that the immunostimulus responsiveness of the predetermined immune cell subset is high. When the localization positive rate of a predetermined immune cell subset contained in whole blood of a subject is the same as a predetermined threshold value, it may be determined that the immunostimulus responsiveness of the predetermined immune cell subset is low or high.

The predetermined threshold value of the localization positive rate is not particularly limited but can be appropriately set. The localization positive rate may also vary depending on the disease, health condition, etc. of a subject. For example, the predetermined threshold value may be empirically set by measuring a sample prepared by mixing whole blood collected from a healthy subject with an immunostimulatory factor *in vitro* by IFC to accumulate data on the localization positive rate.

In addition to immune cells, samples measured by IFC contain particles that are not to be analyzed, such as lysed erythrocyte debris (erythrocyte ghosts). Here, the particles are formed components present in a sample for measurement, and examples thereof include cells, erythrocyte ghosts and platelet aggregation. In particular embodiments using IFC, in order to distinguish immune cells to be analyzed from particles other than the immune cells, transmitted light images and fluorescence images of individual cells are acquired, and based on these images, the immune cells to be analyzed may be counted. For example, the cell size and aspect ratio may be acquired from transmitted light images of individual cells. The total fluorescence signal intensity may be acquired from fluorescence images of individual cells.

As used herein, the term "cell size" is the number of pixels for an image that reflects a cell surrounded by a cell membrane, the region of which cell membrane showing a pixel value lower than a predetermined threshold value is detected in transmitted light images of individual cells. The "aspect ratio" of a cell is the ratio of the length and breadth (number of pixels) of an image that reflects a cell particle in the transmitted light image. For example, particles whose cell size and/or aspect ratio are smaller than a predetermined threshold value can be excluded as debris such as erythrocyte ghosts rather than cells.

As used herein, the term "total fluorescence signal intensity" is the integral value of a pixel value of each pixel constituting an area showing the fluorescence signal in the fluorescence images of individual cells. For example, particles whose total fluorescence signal intensity is smaller than a predetermined threshold value can be excluded as unlabeled cells, that is, cells other than immune cells to be analyzed.

[2. Cell analysis instrument]

The scope of the present invention also includes a cell analysis instrument. A description is made of an embodiment according to this aspect of the present invention with reference to the drawings. The following description is all examples, and the present invention is not limited to this description.

<Embodiment 1 of cell analysis instrument

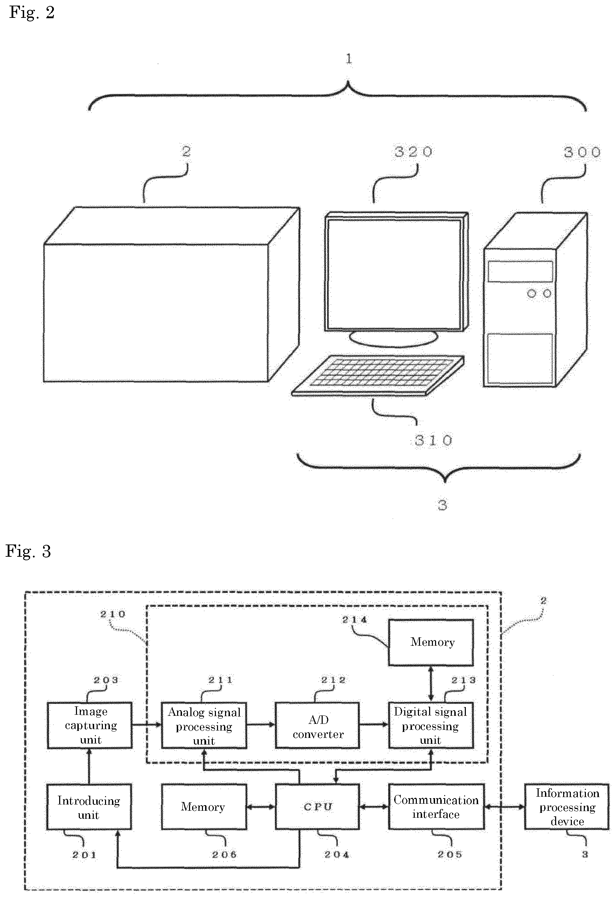

With reference to Fig. 2, a cell analysis instrument 1 includes a measuring device 2 and an information processing device 3. The measuring device 2 optically measures a sample containing immune cells to which a trapping body and a labeling substance are bound by a flow cytometry method. The information processing device 3 analyzes the measurement result obtained by the measuring device 2 and displays the analysis result on a display unit 320. The invention is not limited only to this example, but may be, for example, an instrument in which the measuring device 2 and the information processing device 3 are integrally configured.

With reference to Fig. 3, the measuring device 2 includes an introducing unit 201, an image capturing unit 203, a signal processing unit 210, a CPU 204, a communication interface 205, and a memory 206. The signal processing unit 210 includes an analog signal processing unit 211, an A/D converter 212, a digital signal processing unit 213, and a memory 214.

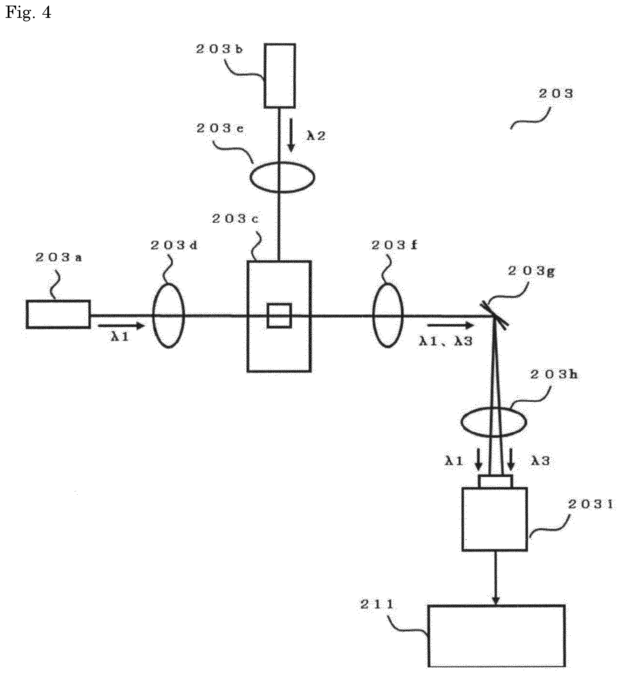

In particular embodiments, the introducing unit 201 includes a container and a pump (not shown). A sample in the container is supplied to a flow cell 203c (see Fig. 4) of the image capturing unit 203 together with a sheath solution by the pump. The introducing unit 201 supplies a sample to the image capturing unit 203 according to the instruction of the CPU 204. In Embodiment 1, the sample to be measured is prepared as follows. By adding an anti-CD28 antibody to human peripheral blood accommodated in the container, followed by centrifugation, immune cells in the peripheral blood are immunostimulated. In this example, a target molecule is CD28 and an anti-CD28 antibody is shared as a trapping body. After fixing cells in the peripheral blood and hemolyzing erythrocytes, the immune cells are labeled with a PE-labeled secondary antibody that binds to the anti-CD28 antibody. However, the present invention is not limited to this example.

The image capturing unit 203 irradiates the sample supplied from the introducing unit 201 with a laser beam, takes images of particles containing the labeled immune cells, and outputs the generated image information on a transmitted light image and a fluorescence image to the analog signal processing unit 211 as an electric signal. The analog signal processing unit 211 amplifies the electric signal output from the image capturing unit 203 according to the instruction of the CPU 204, and outputs the amplified electric signal to the A/D converter 212.

The A/D converter 212 converts the electric signal amplified by the analog signal processing unit 211 into a digital signal and outputs the digital signal to the digital signal processing unit 213. The digital signal processing unit 213 performs predetermined signal processing on the digital signal output from the A/D converter 212 according to the instruction of the CPU 204, to generate the measurement data. The generated measurement data are stored in the memory 214.

The measurement data stored in the memory 214 include a transmitted light image and a fluorescence image based on transmitted light and fluorescence generated when individual particles in the sample pass through the flow cell 203c.

The CPU 204 outputs the measurement data stored in the memory 214 to the communication interface 205. The CPU 204 receives a control signal from the information processing device 3 via the communication interface 205, and controls each unit of the measuring device 2 according to the control signal.

The communication interface 205 transmits the measurement data output from the CPU 204 to the information processing device 3, and receives the control signal output from the information processing device 3. The memory 206 is used as a workspace for the CPU 204.

With reference to Fig. 4, the image capturing unit 203 includes light sources 203a and 203b, a flow cell 203c, condenser lenses 203d and 203e, an objective lens 203f, an optical unit 203g, a condenser lens 203h, and a camera 203i.

In particular embodiments, the light source 203a is a semiconductor laser. The light emitted from the light source 203a is a laser beam having a wavelength of $\lambda 1$. The condenser lens 203d condenses the light emitted from the light source 203a and guides the condensed light to the sample in the flow cell 203c. Individual particles in the sample passing through the inside of the flow cell 203c are irradiated with the light having a wavelength of $\lambda 1$ emitted from the light source 203a, whereby transmitted light having a wavelength of $\lambda 1$ is generated from the particles.

The light source 203b is a semiconductor laser light source. The light emitted from the light source 203b is a laser beam having a wavelength of λ2. In particular embodiments, the wavelength λ2 is about 488 nm. The condenser lens 203e condenses the light emitted from the light source 203b and guides the condensed light to the sample in the flow cell 203c. Individual particles in the sample passing through the inside of the flow cell 203c are irradiated with the light having a wavelength of λ2 emitted from the light source 203b. When PE-labeled immune cells are irradiated with light, PE emits fluorescence having a wavelength of λ3.

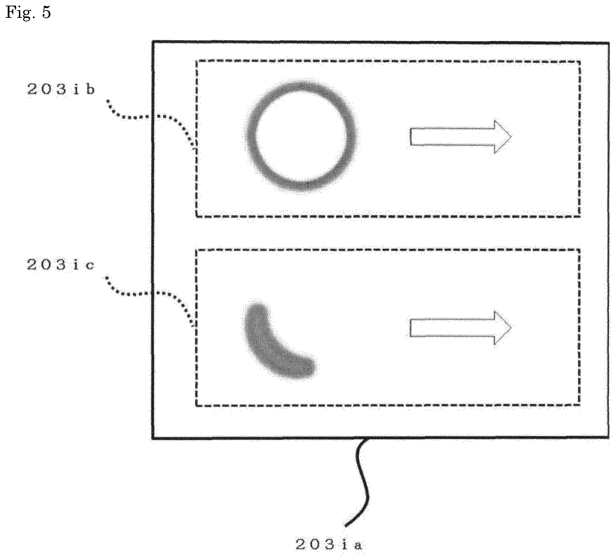

The objective lens 203f condenses transmitted light having a wavelength of λ1 and fluorescence having a wavelength of λ3. The optical unit 203g has a configuration in which two dichroic mirrors are combined. The two dichroic mirrors of the optical unit 203g reflect the transmitted light having a wavelength of λ1 and the fluorescence having a wavelength of λ3 at different angles, which are separated on the light receiving surface 203ia (see Fig. 5) of the camera 203i described later. The condenser lens 203h condenses the transmitted light having a wavelength of λ1 and the fluorescence having a wavelength of λ3. The camera 203i receives the transmitted light having a wavelength of λ1 and the fluorescence having a wavelength of λ3, and outputs the image information on the sample in the flow cell 203c as an electric signal to the analog signal processing unit 211. The camera 203i may be a time delay integration (TDI) camera. By using a TDI camera, more accurate image information can be acquired.

As shown in Fig. 5, the camera 203i receives the transmitted light having a wavelength of λ1 and the fluorescence having a wavelength of λ3 in the light receiving regions 203ib and 203ic on the light receiving surface 203ia, respectively. The light receiving surface 203ia is a light receiving surface of an image sensor such as a CMOS image sensor arranged on the camera 203i. The position of the light emitted to the light receiving surface 203ia moves within each of the light receiving regions 203ib and 203ic, following the moving of the sample in the flow cell 203c, as shown by the arrows. In this way, the two lights are separated on the light receiving surface 203ia by the optical unit 203g, so that the CPU 204 can extract a signal corresponding to each light from the image signal output by the camera 203i.

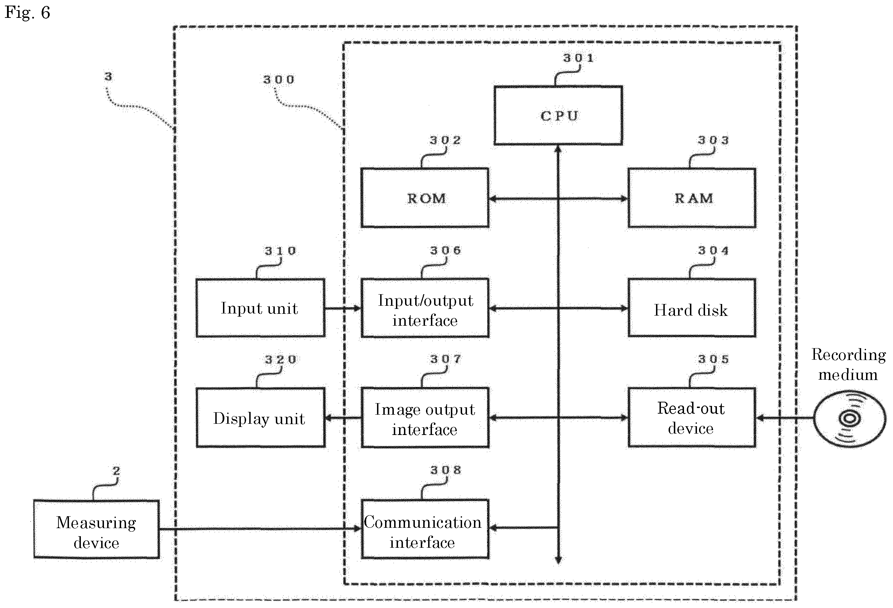

With reference to Fig. 6, the information processing device 3 includes a main body 300, an input unit 310 and a display unit 320. The main body 300 includes a central processing unit (CPU) 301, a read only memory (ROM) 302, a random access memory (RAM) 303, a hard disk 304, a read-out device 305, an input/output interface 306, an image output interface 307, and a communication interface 308.

The CPU 301 executes a computer program stored in the ROM 302 and a computer program loaded in the RAM 303. The computer program stored in the ROM 302 includes a basic input output system (BIOS). The RAM 303 is used to read out the computer programs recorded in the ROM 302 and the hard disk 304. The RAM 303 is also used as a workspace for the CPU 301 when executing these computer programs.

The hard disk 304 stores various computer programs such as an operating system (OS) and an application program to be executed by the CPU 301, and data used for executing the computer programs. In addition, the hard disk 304 stores the measurement data received from the measuring device 2.

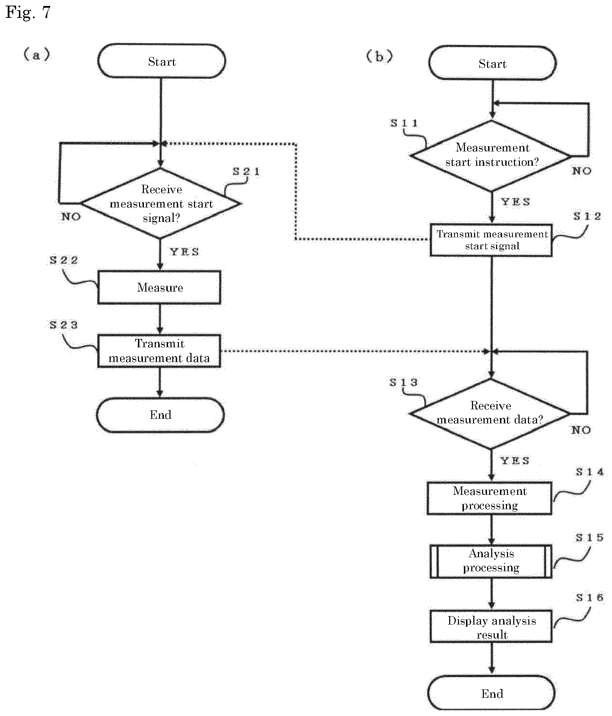

The hard disk 304 stores a program for measuring parameters for analysis including the number, cell size, aspect ratio, fluorescence signal area value and total fluorescence signal intensity of immune cells contained in the sample based on the measurement data to analyze the sample, and a display program for displaying the analysis result on the display unit 320. By storing these programs, analysis processing and display processing described later are performed. That is, the CPU 301 is provided with a function of executing the processing of Fig. 7b described later by these programs.

The read-out device 305 is composed of a CD-ROM drive, a DVD-ROM drive, a USB port, an SD card reader, a CF card reader, a memory stick reader, a solid state drive, a flexible disk drive, or the like. The read-out device 305 can read out computer programs and data recorded in a portable recording medium such as a CD or DVD.

An input unit 310 composed of a mouse, a keyboard and the like is connected to the input/output interface 306, and a user inputs an instruction to the information processing device 3 using the input unit 310. The image output interface 307 is connected to a display unit 320 composed of a display or the like, and outputs a video signal corresponding to the image data to the display unit 320. The display unit 320 displays an image based on the input video signal.

The information processing device 3 can receive the measurement data transmitted from the measuring device 2 by the communication interface 308. The received measurement data are stored in the hard disk 304.

Fig. 7 is a flowchart showing the control of the CPU 204 of the measuring device 2 and the CPU 301 of the information processing device 3. Fig. 7a is a flowchart showing the control processing by the CPU 204 of the measuring device 2, and Fig. 7b is a flowchart showing the control processing by the CPU 301 of the information processing device 3.

Reference is made to Fig. 7b for the control processing of the CPU 301 of the information processing device 3. When a user inputs a measurement start instruction via the input unit 310 (step S11: YES), the CPU 301 transmits a measurement start signal to the measuring device 2 (step S12). Subsequently, the CPU 301 determines whether or not the measurement data have been received (step S13). When no measurement data have been received (step S13: NO), processing is in a standby state.

Reference is made to Fig. 7a for the control processing of the CPU 204 of the measuring device 2. When the CPU 204 receives the measurement start signal from the information processing device 3 (step S21: YES), the CPU 204 measures the sample (step S22). In the measurement processing (step S22), the sample containing labeled immune cells is supplied from the introducing unit 201 to the flow cell 203c together with a sheath solution, and a flow of the sample enveloped with the sheath solution is formed in the flow cell 203c. The formed sample flow is irradiated with laser beams from the light sources 203a and 203b, so that a beam spot is formed in the flow cell 203c. When individual particles in the sample pass through the beam spot, transmitted light and fluorescence are generated. The generated transmitted light and fluorescence are captured by the camera 203i and converted into electric signals.

These electric signals are converted into digital signals by the A/D converter 212, and signal processing is performed by the digital signal processing unit 213. As a result, measurement data including a transmitted light image and a fluorescence image are obtained for each complex that has passed through the flow cell 203c. The measurement data are stored in the memory 214. When the measurement of the sample is completed, the CPU 204 transmits the measurement data generated by the measurement processing to the information processing device 3 (step S23), and ends the process.

With reference to Fig. 7b again, when the CPU 301 of the information processing device 3 receives the measurement data from the measuring device 2 (step S13: YES), the CPU 301 stores the measurement data in the hard disk 304 and performs the measurement processing based on the measurement data to yield parameters for analysis (step S14). Then, the CPU 301 performs the analysis processing using the parameters for analysis (step S15). Subsequently, the CPU 301 displays the analysis result acquired in S14 on the display unit 320 (step S16). After that, the processing ends.

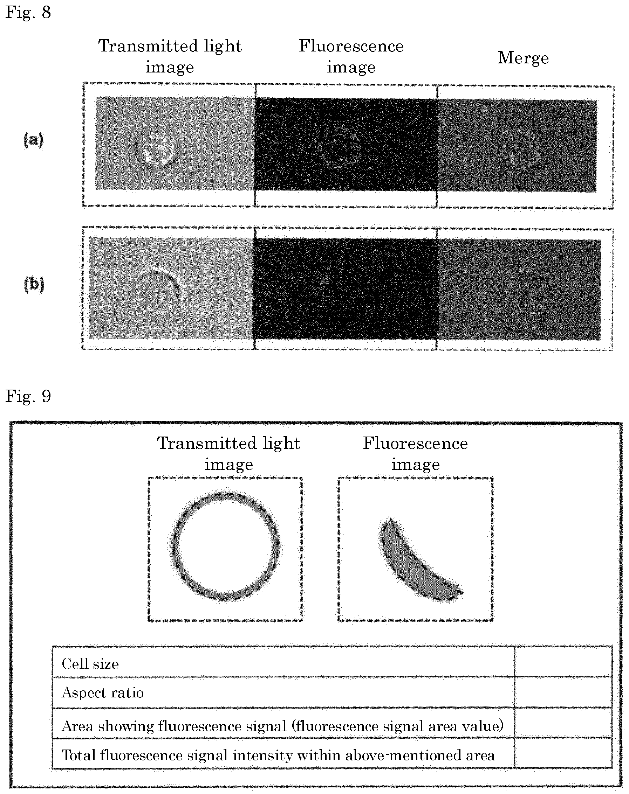

With reference to Fig. 8 to Fig. 10, a further description is made of an example of the analysis processing performed by the CPU 301 of the information processing device 3 based on the measurement data (step S14 in Fig. 7b). Fig. 8 shows a transmitted light image, a fluorescence image (CD28), and a merged image thereof (MERGE) of the measurement data obtained by measuring the labeled immune cells. As shown in the fluorescence image of Fig. 8a, CD28 tends to be uniformly distributed on the cell membrane of immune cells in the absence of immunostimulation. As shown in the fluorescence image of Fig. 8b, CD28 tends to localize on a part of the cell membrane of immune cells when immunostimulated. Without wishing to be bound by any theory, it is believed that the part of the cell membrane where CD28 localizes represents an immunological synapse.

The CPU 301 performs the measurement processing based on the transmitted light images and the fluorescence images of all the particles captured in step S22 of Fig. 7a to yield parameters for analysis (step S14 in Fig. 7b). Examples of the parameters for analysis include, but are not limited to, cell size (number of pixels), cell aspect ratio, area showing the fluorescence signal in a cell (fluorescence signal area value: number of pixels), total fluorescence signal intensity within the area, and the ratio between them. Fig. 9 shows a schematic view of the measurement processing (step S14 in Fig. 7b) of the transmitted light image and the fluorescence image (see Fig. 8b) shown by the CPU 301 of the information processing device 3. The obtained measurement data are stored in the hard disk 304. The meanings of the respective terms of cell size, aspect ratio, fluorescence signal area value, and total fluorescence signal intensity are as described above.

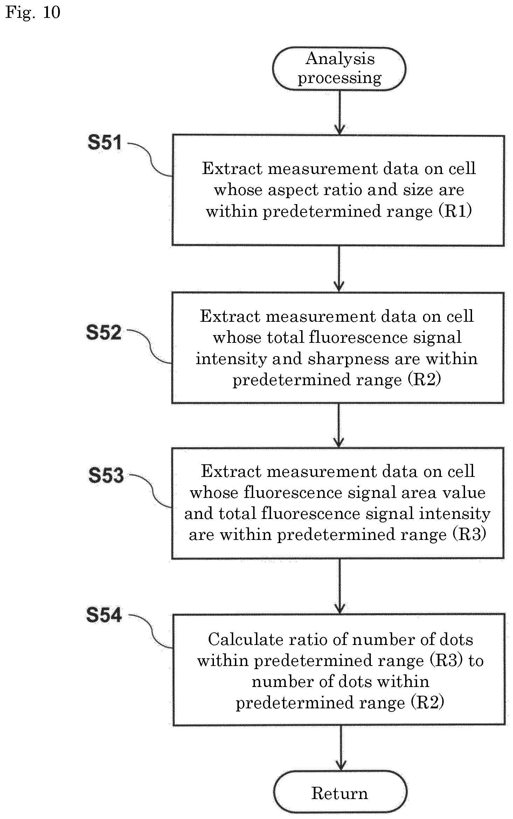

Fig. 10 is a flowchart showing the analysis processing in step S15 of the CPU 301 of the information processing device 3 (see Fig. 7b). The CPU 301 creates a two-dimensional histogram (2D scattergram) in which the parameters for analysis measured in step S14 in Fig. 7b are each assigned on the X-axis and Y-axis, and extracts and analyzes the measurement data on cells corresponding to dots existing within a predetermined range. The analysis processing described below is only an example, and different analysis processing may be used in the invention.

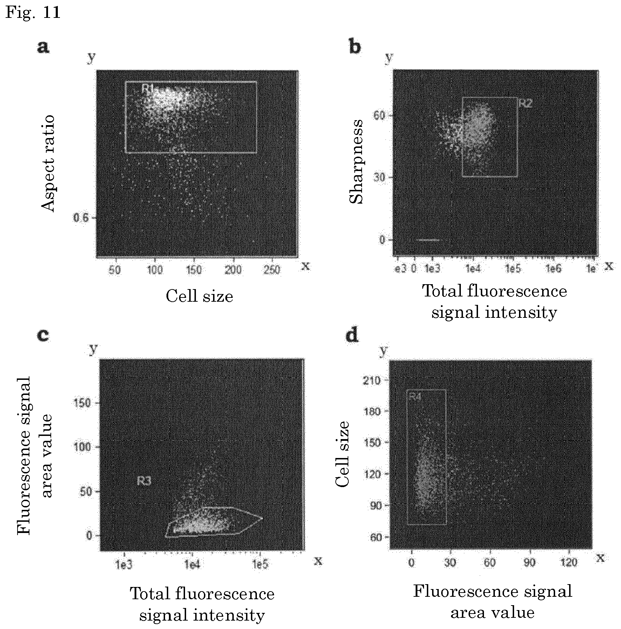

In step S51, the CPU 301 creates a 2D scattergram in which the cell size is assigned on the X-axis and the cell aspect ratio is assigned on the Y-axis (see Fig. 11a). Each dot displayed on the 2D scattergram corresponds to each particle measured. The CPU 301 extracts the measurement data on a cell within a predetermined region (hereinafter referred to as "predetermined range (R1)") displayed as R1 in the 2D scattergram. The predetermined range (R1) is a region gated within a predetermined range that reflects the size of a single cell in the cell size (X-axis), and gated within a predetermined range that reflects the existence of a cell alone in the aspect ratio (Y-axis). This excludes data derived from debris such as erythrocyte ghosts that are smaller than the cell and aggregates of cells that are larger than a single cell.

In particular embodiments, the dots displayed on the 2D scattergram are linked to the measurement data on the individual particles measured in step S22 in Fig. 7a. When a dot displayed on the 2D scattergram is specified by a user, the CPU 301 reads out the measurement data (transmitted light image and/or fluorescence image) of a particle corresponding to the dot from the hard disk 304, and displays the measurement data on the display unit 320.

In step S52, the CPU 301 creates a 2D scattergram of the measurement data on cells extracted in step S51 in which the total fluorescence signal intensity is assigned on the X-axis and the sharpness of fluorescence image (image sharpness) is assigned on the Y-axis (Fig. 11b). The CPU 301 further extracts the measurement data on a cell within a predetermined region (hereinafter referred to as "predetermined range (R2)") displayed as R2 in the 2D scattergram. The range (R2) is a region gated within a range that reflects a predetermined CD28 expression level in the total fluorescence signal intensity (X-axis), and gated within a range that reflects the sharpness of a predetermined image in the sharpness (Y-axis). This extracts data on cells expressing a predetermined amount of CD28, and excludes data on cells not expressing a predetermined amount of CD28.

In this example, the 2D scattergram shown in Fig. 11b is created, but it is not necessary to create this 2D scattergram. For example, data on cells expressing a predetermined amount of CD28 may be extracted based solely on the total fluorescence signal intensity. Specifically, data on cells expressing a predetermined amount of CD28 are extracted by extracting data having a total fluorescence signal intensity higher than a predetermined value from the measurement data on cells extracted in step S51.

In step S53, the CPU 301 creates a 2D scattergram of the measurement data extracted in step S52 in which the total fluorescence signal intensity of cells is assigned on the X-axis and the area showing the fluorescence signal is assigned on the Y-axis (Fig. 11c). The CPU 301 counts the number of measurement data in a predetermined region (hereinafter referred to as "predetermined range (R3)") displayed as R3 in the 2D scattergram. The range (R3) is a region gated within a range that reflects a predetermined CD28 expression level in the total fluorescence signal intensity of the cells (X-axis), and gated within a predetermined range that reflects the localization of CD28 in the fluorescence signal area value (Y-axis). In particular embodiments, cells corresponding to dots within the range (R3) tend to have CD28 localized. Accordingly, immune cells on which CD28 localizes are counted by counting the dots within the range (R3).

In step S54, the CPU 301 may count the number of dots shown throughout the 2D scattergram in Fig. 11c (corresponding to the number of dots within the range (R2) in Fig. 11b) to calculate the ratio of the number of dots within the range (R3) to the number of dots within the range (R2) ([the number of dots within the range (R3)]/[the number of dots within the range (R2)]). As a result, the ratio of immune cells on which CD28 localizes to immune cells expressing a predetermined amount of CD28 is calculated. The analysis results such as the number of dots and the ratio within each range obtained by the analysis processing are displayed on the display unit 320.

<Embodiment 2 of cell analysis instrument

Instead of step S53 of the analysis processing in Embodiment 1, the CPU 301 may create a 2D scattergram of the measurement data extracted in step S52 in which the fluorescence signal area value is assigned on the X-axis and the cell size is assigned on the Y-axis (Fig. 11d). The CPU 301 may count the number of measurement data in a predetermined region (hereinafter referred to as "predetermined range (R4)") displayed as R4 in the 2D scattergram. The range (R4) is gated within a predetermined range that reflects the localization of CD28 in the fluorescence signal area value (X-axis), and gated within a predetermined range that reflects the size of a single cell in the cell size (Y-axis). The number of immune cells responsive to immunostimulation is counted by counting the measurement data within the range (R4). In the embodiment, cells corresponding to dots within the range (R4) tend to have CD28 localized. Accordingly, immune cells on which CD28 localizes are counted by counting the dots within the range (R4).

Instead of step S54 of the analysis processing in Embodiment 1, the CPU 301 may count the number of dots shown throughout the 2D scattergram in Fig. 11d (corresponding to the number of dots within the range (R2) in Fig. 11b) to calculate the ratio of the number of dots within the range (R4) to the number of dots within the range (R2) ([the number of dots within the range (R4)]/[the number of dots within the range (R2)]). As a result, the ratio of immune cells on which CD28 localizes to immune cells expressing a predetermined amount of CD28 is calculated. The analysis results such as the number of dots and the ratio within each range obtained by the analysis processing are displayed on the display unit 320.

<Embodiment 3 of cell analysis instrument

In the analysis of the measurement data, the localization of target molecule on immune cells may be analyzed without using a 2D scattergram. For example, instead of step S53 of the analysis processing in Embodiment 1, the CPU 301 may create a histogram of the measurement data on cells extracted in step S52 in which the class value of the fluorescence signal area value is assigned on the X axis and the frequency is assigned on the Y-axis, and display the histogram on the display unit 320. Instead of step S54 of the analysis processing in Embodiment 1, the CPU 301 may calculate at least one of the mean, median and mode values of the fluorescence signal area values of the measurement data extracted in step S52 from the histogram. Further analysis may be performed using the obtained values. For example, the mode value of the fluorescence signal area values obtained from a sample derived from whole blood of a subject may be compared with the standardized mode value of the fluorescence signal area values obtained from a sample derived from whole blood of a healthy subject. In this analysis, when the class value indicating the above-mentioned mode value of a subject is smaller than the class value indicating the standardized mode value of a healthy subject, it can be determined that more CD28 localizes on immune cells in whole blood of the subject than that on immune cells in whole blood of the healthy subject.

<Embodiment 4 of cell analysis instrument

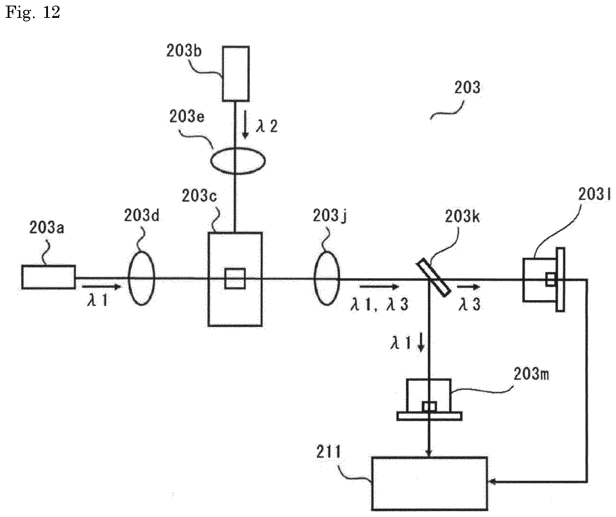

In the analysis processing of Embodiments 1 to 3, the analysis based on the captured image data is illustrated, but the localization of target molecule on immune cells can be analyzed from the measurement data other than the image data. As a modification of the image capturing unit 203, with reference to Fig. 12, for example, the image capturing unit 203 may be a detection unit that detects an optical signal, including light sources 203a and 203b, a flow cell 203c, condenser lenses 203d, 203e and 203j, a dichroic mirror 203k, a fluorescence light receiving unit 2031, and a forward scattered light receiving unit 203m.

The light source 203b is a semiconductor laser light source. The light emitted from the light source 203b is a laser beam having a wavelength of λ2. In the embodiment, the wavelength λ2 is about 488 nm. The condenser lens 203e condenses the light emitted from the light source 203b and guides the condensed light to the sample in the flow cell 203c. Individual particles in the sample passing through the inside of the flow cell 203c are irradiated with the light having a wavelength of λ2 emitted from the light source 203b. When PE-labeled immune cells are irradiated with light, PE emits fluorescence having a wavelength of λ3. The condenser lens 203j condenses the transmitted light having a wavelength of λ1 and the fluorescence having a wavelength of λ3. The forward scattered light having a wavelength of λ1 is reflected by the dichroic mirror 203k and received by the forward scattered light receiving unit 203m. The fluorescence having a wavelength of λ3 passes through the dichroic mirror 203k and is received by the fluorescence light receiving unit 2031. An avalanche photodiode, photodiode or photomultiplier tube can be used as the fluorescence light receiving unit 2031 and the forward scattered light receiving unit 203m.

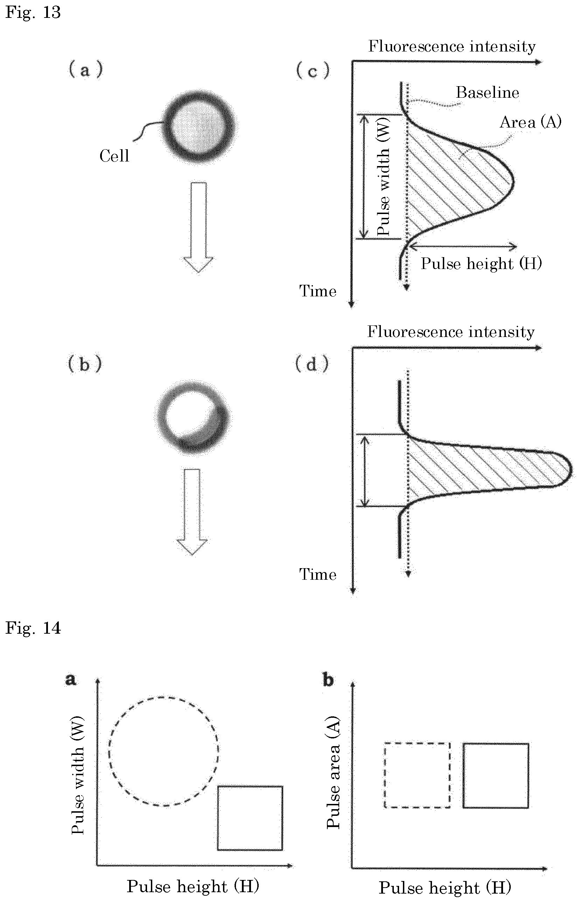

In this example, the optical signals obtained in step S22 in Fig. 7a are a forward scattered light signal and a fluorescence signal. In addition, in step S14 in Fig. 7b, "pulse width (W)", "pulse area (A)" and "pulse height (H)" are obtained as parameters for analysis. A description is made of the parameters for analysis with reference to Fig. 13.

Fig. 13 is schematic diagrams (Figs. 13c and 13d) each showing a fluorescence signal detected while a cell on which CD28 is uniformly distributed on the cell membrane (Fig. 13a) and a cell on which CD28 localizes on the cell membrane (Fig. 13b) pass through the beam spot in the direction of the arrow.

As used herein, in a pulse of fluorescence intensity as shown in Figs. 13c and 13d, the "pulse width (W)" refers to a time when the fluorescence signal is obtained beyond the threshold baseline, and the "pulse height (H)" refers to a fluorescence intensity when the peak of the pulse is shown in the fluorescence intensity. The "pulse area (A)" refers to an area between the baseline and the fluorescence signal intensity curve.

In this example, the same amount of CD28 is expressed on the cell membrane for the cell of Fig. 13a and the cell of Fig. 13b, so that the pulse areas (A) in Figs. 13c and 13d have an equal value. Here, the cells differ from each other in pulse width (W) and pulse height (H) based on the distribution pattern of CD28. In the case where CD28 localizes on the cell membrane (Fig. 13b), the pulse width (W) is smaller, while the pulse height (H) is higher (Figs. 13c and 13d), compared to the case where CD28 is uniformly distributed on the cell membrane (Fig. 13a).

Fig. 14 is a schematic view in the case where a plurality of immune cells are measured to create a 2D scattergram in Embodiment 4. Fig. 14a shows a 2D scattergram in which the pulse height (H) is assigned on the X-axis and the pulse width (W) is assigned on the Y-axis, and Fig. 14b is a 2D scattergram in which the pulse height (H) is assigned on the X-axis and the pulse area (A) is assigned on the Y-axis.

As described above, in the case where co-stimulatory molecules are uniformly distributed on the cell membrane, the pulse width (W) tends to increase, while the pulse height (H) tends to be low, compared to the case where the co-stimulatory molecules localize on the cell membrane. Accordingly, in the 2D scattergram shown in Fig. 14a, the data in the region surrounded by the circle of the dashed line are data that reflect cells having the cell membrane on which CD28 is uniformly distributed. On the other hand, the data in the region surrounded by the square of the solid line tends to be data that reflect cells having the cell membrane on which CD28 localizes. In the 2D scattergram shown in Fig. 14b, the data in the region surrounded by the square of the dashed line are data that reflect cells having the cell membrane on which CD28 is uniformly distributed. On the other hand, the data in the region surrounded by the square of the solid line tends to be data that reflect cells having the cell membrane on which CD28 localizes.

As illustrated in Embodiment 4, it is understood that the localization of target molecule on immune cells can be analyzed not only by the analysis processing based on captured image data, but also by the analysis processing based on an optical signal as described above.

The parameters for analysis and analysis processing shown here are only examples, and different parameters for analysis may be measured from the measurement data, or different analysis processing may be performed on the measured parameters for analysis. For example, the pulse area is not limited to the time integral value, but may be an approximate value as long as it is a value that reflects the area under the time curve of pulse. The pulse area may be a product of the pulse width and the peak height, or may be an area of a triangle obtained from the pulse width and the peak height. In addition, in the form of measuring the time integral value, the base does not have to be the baseline, but can be appropriately set. For example, a value beyond a predetermined threshold value from the baseline may be used as the base.

Hereinafter, a detailed description is made of the present invention with reference to Examples, but the present invention is not limited to the Examples.

EXAMPLES

Example 1: Measurement of immunostimulus responsiveness of immune cells in whole blood

By adding an anti-CD28 antibody as an immunostimulatory factor to whole blood (peripheral blood) collected from a subject, immune cells (T cells) in the whole blood were stimulated, whereby information on the localization of CD28 on the immune cells was acquired. As a control, measurement was performed for immune cells in whole blood to which no immunostimulatory factor was added.

1. Measurement

(1) Measurement of stimulated immune cells

Whole blood was collected from 5 subjects (healthy volunteers). The whole blood (200 $\mu$L) of each subject was collected in a tube, and a mouse anti-human CD28 antibody (# 302914 Clone: CD28.2: BioLegend) was added, followed by gentle stirring. The whole blood containing the antibody was centrifuged at 200 $\times$g for 2 minutes at room temperature to form a buffy coat. By allowing the buffy coat to stand in a $CO_2$ incubator (37°C) for about 2 to 120 minutes, immune cells to be analyzed were brought into contact with other leukocytes, followed by addition of a fixing solution containing 4% PFA to fix the cell membrane of the immune cells. The time from the addition of the anti-human CD28 antibody to the addition of 4% PFA was about 5 to 125 minutes. Next, a hemolytic agent (0.4 to 0.8% ammonium chloride) was added to perform hemolytic treatment. Then, cells and cell nuclei were stained with a PE-labeled goat anti-mouse IgG antibody (# 405307: BioLegend), an APC-labeled mouse anti-human CD3 antibody (# 300411 Clone: UCHT1: BioLegend) and Hoechst (H342: Dojindo Laboratories). Stained cells were suspended in 1% BSA/PBS, and measured using an imaging flow cytometer (ImageStreamX Mark II Imaging Flow Cytometer: Amnis).

(2) Measurement of unstimulated immune cells

Immune cells contained in the whole blood (200 $\mu$L) of the above-mentioned healthy volunteers were treated in the same manner as in (1) except that the mouse anti-human CD28 antibody was not added, and measured using IFC.

2. Data analysis

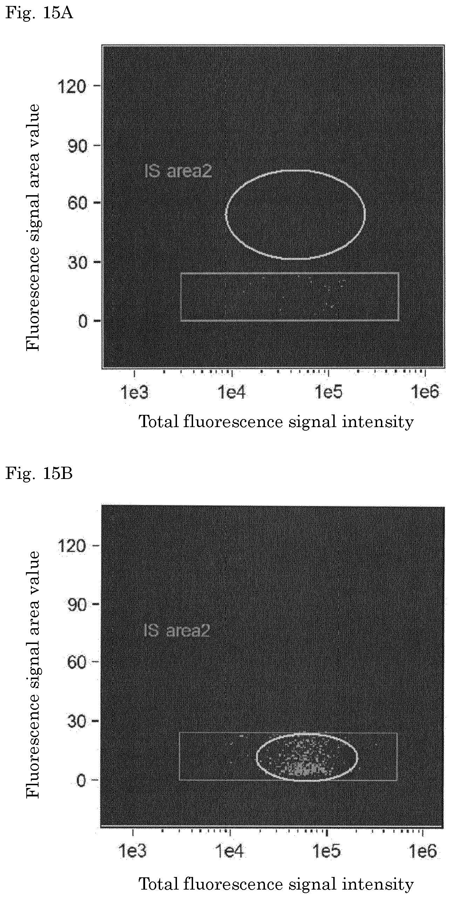

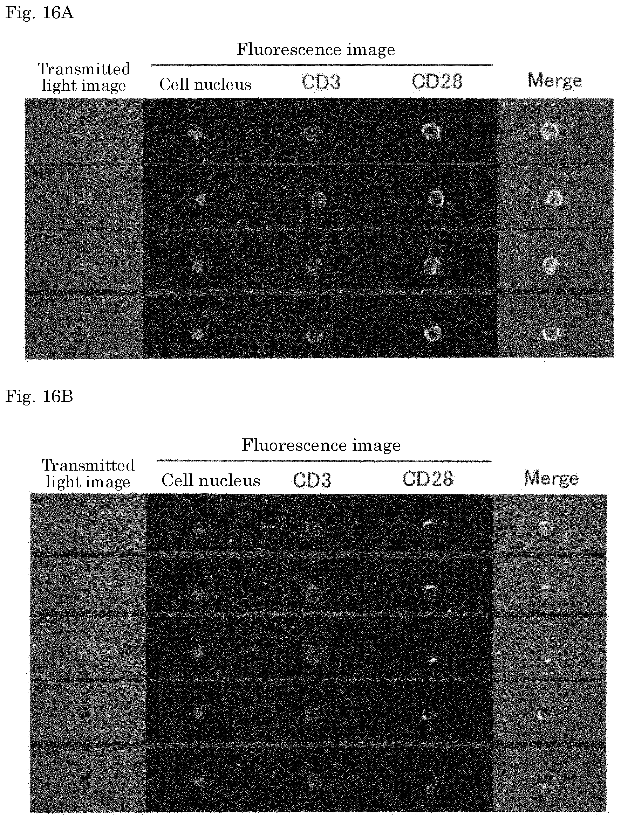

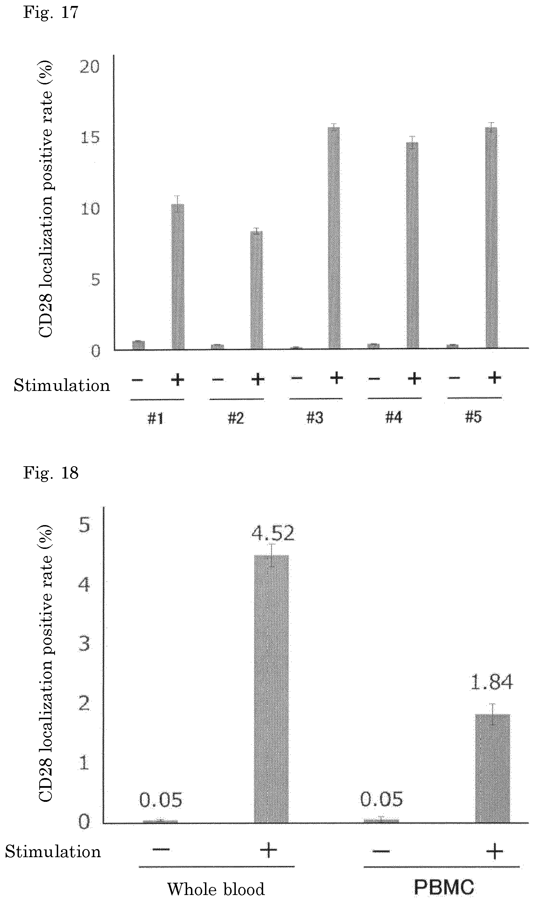

Multi-parameter analysis was performed on the measurement data on the whole blood containing stimulated immune cells and the measurement data on the whole blood containing unstimulated immune cells obtained by measurement by IFC. Briefly, image analysis was performed based on transmitted light images and fluorescence images (CD28) of individual cells obtained by IFC, whereby cell size, cell aspect ratio, fluorescence signal area value, and total fluorescence signal intensity were acquired. First, based on the transmitted light image data on individual cells, a 2D scattergram was created in which the cell size was assigned on the X-axis and the cell aspect ratio was assigned on the cells on the Y-axis (not shown). In order to exclude the measurement data on debris such as erythrocyte ghosts, dots within a predetermined range (Cells) of the 2D scattergram were selected, and the measurement data corresponding to each selected dot were extracted as the measurement data on a cell.
Then, based on the measurement data within the range (Cells), a 2D scattergram was created in which the total fluorescence signal intensity was assigned on the X-axis and the fluorescence signal area value was assigned on the Y-axis (not shown). In the 2D scattergram, dots within the range (T cells) where the total fluorescence signal intensity was higher than or equal to a predetermined value were selected. The measurement data corresponding to each selected dot were extracted as the measurement data on an immune cell (CD28-positive T cell). For the extracted measurement data, a 2D scattergram was created in which the total fluorescence signal intensity was assigned on the X-axis and the fluorescence signal area value was assigned on the Y-axis. Then, from the measurement data within the range (T cells), the measurement data on a cell population having a low fluorescence signal area value were extracted. Specifically, in the created 2D scattergram, dots within a predetermined range (IS area 2) were selected, and measurement data corresponding to each selected dot were extracted as the measurement data on an immunostimulus responsive T cell. IS is an abbreviation for immunological synapse.
An example of the 2D scattergram created based on the measurement data within the range (T cells) is shown in Fig. 15A (unstimulated immune cells) and Fig. 15B (stimulated immune cells). In Figs. 15A and 15B, the region surrounded by a rectangle is the range (IS area 2). In addition, in Figs. 15A and 15B, an example of the image of a cell corresponding to each dot within the region surrounded by an ellipse is shown in Figs. 16A and 16B. Dots within the range (T cells) and range (IS area 2) were counted, and the ratio of T cells on which CD28 localized to T cells in whole blood (CD28 localization positive rate) was calculated from the following formula. The CD28 localization positive rate of T cells for each subject is shown in Fig. 17.

$$[\text{CD28 localization positive rate (\%)}] = \{[\text{number of dots within range (IS area 2)}]/[\text{number of dots within range (T cells)}]\} \times 100$$

3. Result and discussion

As can be seen from Fig. 16A, for T cells that had not been immunostimulated with the anti-CD28 antibody, the CD28 molecules were uniformly distributed on the cell membrane. On the other hand, as can be seen from Fig. 16B, for the immunostimulated T cells, the CD28 molecules localized on a part of the cell membrane. It is believed that the portion showing this localization in the fluorescence image of CD28 represents a formed immunological synapse. In the 2D scattergrams of Figs. 15A and 15B, cells showing low fluorescence signal area values tend to form an immunological synapse. Accordingly, the region where the fluorescence signal area value is small is gated, whereby a region (range (IS area 2)) in which immune cells exhibiting immunostimulus responsiveness appear is shown. Comparing Fig. 15A (unstimulated immune cells) with Fig. 15B (stimulated immune cells), it can be seen that cells are prominently appearing in the gated region (range (IS area 2) in Fig. 15B.

From Fig. 17, the CD28 localization positive rate showed very low value in the whole blood collected from any subject without stimulation. On the other hand, the CD28 localization positive rate increased in the case with stimulation, and a remarkable difference was observed as compared with the case without stimulation. From the above, it has been indicated that the method of the invention using whole blood as a sample can detect immune cells exhibiting immunostimulus responsiveness (immune cells exhibiting IS-forming ability). It has also been indicated that the immunostimulus responsiveness (IS-forming ability) of immune cells can be evaluated based on the detection results.

Example 2: Comparison of method of the invention using whole blood with conventional method using isolated PBMC (1)

The method of the invention using whole blood as a sample was compared with a conventional method using PBMCs (peripheral blood mononuclear cells) isolated from whole blood as a sample using the CD28 localization positive rate as an index.

1. Measurement

(1) Measurement of immune cells in whole blood

Whole blood was collected from healthy volunteers. Using a part of the collected whole blood (200 $\mu$L), immune cells stimulated with the anti-human CD28 antibody and unstimulated immune cells were measured in the same manner as in Example 1.

(2) Measurement of immune cells in PBMCs

A portion (200 $\mu$L) of the whole blood was taken, followed by density gradient centrifugation using Ficoll to isolate PBMCs. The isolated PBMCs were collected in a tube, and the same anti-human CD28 antibody as in Example 1 was added, followed by centrifugation at 200 $\times$ g for 2 minutes at room temperature to settle the cells. By allowing the cells to stand in a $CO_2$ incubator (37°C) for about 2 to 120 minutes, immune cells were brought into contact with the wall surface of the tube, followed by addition of a fixing solution containing 4% PFA to fix the cell membrane of the immune cells. Next, the same hemolytic agent as in Example 1 was added to perform hemolysis treatment. The cells and cell nuclei were then stained with the same PE-labeled goat anti-mouse IgG antibody, APC-labeled mouse anti-human CD3 antibody and Hoechst as in Example 1. Stained cells were measured by IFC in the same manner as in Example 1. Cells that had not been immunostimulated prepared by treating PBMCs isolated from the whole blood in the same manner as above except that the mouse anti-human CD28 antibody had not been added were measured using IFC.

2. Data analysis

Multiple parameter analysis of the data obtained by IFC measurement was performed in the same manner as in Example 1. The ratio of T cells on which CD28 localized to T cells in whole blood or isolated PBMCs (CD28 localization positive rate) was calculated. The results are shown in Fig. 18.

3. Result and discussion

From Fig. 18, in the case without stimulation, the CD28 localization positive rates of T cells in PBMCs and whole blood showed similar values. On the other hand, in the case with stimulation, the CD28 localization positive rate of T cells in PBMCs showed prominently lower value than that in whole blood. This is believed to suggest changes in immune cell quality and function due to the step of separating and isolating PBMCs from whole blood. In the method of the invention, since the step of separating and isolating PBMCs is not performed, the influence on the quality and function due to the step is eliminated. Therefore, it has been indicated that the method of the invention improves the measurement accuracy as compared with a conventional method using PBMCs.

Example 3: Comparison of method of invention using whole blood with conventional method using isolated PBMC (2)

The method of the invention using whole blood as a sample was compared with a conventional method using PBMCs (peripheral blood mononuclear cells) isolated from whole blood as a sample using a noise signal as an index.

1. Measurement and data analysis

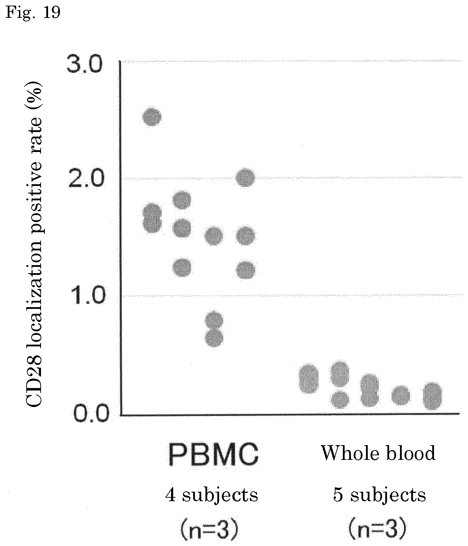

In the same manner as in Example 1, using whole blood collected from 5 subjects, T cells in the whole blood without adding the anti-CD28 antibody were measured to calculate the CD28 localization positive rate. In addition, in the same manner as in Example 2, PBMCs were isolated from whole blood collected from 4 subjects, and T cells in the PBMCs without adding the anti-CD28 antibody were measured to calculate the CD28 localization positive rate. The results are shown in Fig. 19.

2. Result and discussion

From Fig. 19, comparing the CD28 localization positive rate of T cells in whole blood and PBMCs under the condition without stimulation, data variation and a noise signal were observed in the PMBCs. This is believed to suggest that the step of separating and isolating PBMCs from whole blood affects the quality and function of immune cells. On the other hand, in the method of the invention using whole blood, since the step of separating and isolating PBMCs is not performed, the variation in data and the noise signal due to the step was reduced. Therefore, it has been indicated that the method of the invention improves the measurement accuracy as compared with a conventional method using PBMCs.

Example 4: Effect of immunostimulation in whole blood on localization and measurement of target molecule

Using a T cell clone as the immune cells to be analyzed, measurement was performed by the method of the invention and a conventional method to compare the CD28 localization positive rates of the T cell clone.

1. Preparation of T cell clone

In a Yssel's medium (400102: Gemini Bio-Products) containing 1 $\mu$g/mL LEAF(trademark) Purified Anti-Human CD3 Antibody (317315: Biolegend), 10 ng/mL recombinant human IL-2 (rhIL-2) (202-IL-500: R&D systems), 0.2 $\mu$g/mL Phytohaemagglutinin-L (PHA) (L4144-5MG: SIGMA) and 2% human serum (H4522-100ML: SIGMA) were seeded 5 $\times$ $10^6$ CD4 positive cells (ST-70026: Stemcell Technologies) and CD8 positive cells (0508-100: Funakoshi Co., Ltd., PB08C-1: Cosmo Bio Co., Ltd.), followed by co-culture with 1 $\times$ $10^6$ PBMCs and 1 $\times$ $10^4$ JY cells for 14 days. After 14 days, T cells were seeded on a 96-well plate by limiting dilution to 0.3, 1.0 and 3.0 cells/well, and co-cultured with 1 $\times$ $10^4$ PBMCs until colonies were formed. T cells were collected from the wells in which colonies had been formed to establish the T cell clone.

2. Measurement

(1) Measurement of T cell clone by method of the invention

The Hoechst-labeled T cell clone (5 $\times$ $10^5$ cells/100 $\mu$L) was added to whole blood (200 $\mu$L) collected from healthy volunteers, and the same anti-human CD28 antibody as in Example 1 was added. A buffy coat was formed in

**EP 3 889 601 A1**

the same manner as in Example 1. By allowing the buffy coat to stand in a $CO_2$ incubator (37°C) for about 2 to 120 minutes, immune cells containing the T cell clone were brought into contact with other leukocytes, followed by addition of a fixing solution containing 4% PFA to fix the cell membrane of the immune cells. Next, the same hemolytic agent as in Example 1 was added to perform hemolysis treatment. The cells were then stained with the same PE-labeled goat anti-mouse IgG antibody and APC-labeled mouse anti-human CD3 antibody as in Example 1. Stained cells were measured by IFC in the same manner as in Example 1. The T cell clone added to whole blood that had not been immunostimulated prepared in the same manner as above except that the mouse anti-human CD28 antibody had not been added was measured by IFC.

(2) Measurement of T cell clone by conventional method

The Hoechst-labeled T cell clone ($5 \times 10^5$ cells/100 µL) was seeded on a 48-well plate, and the same anti-human CD28 antibody as in Example 1 was added. The cells were allowed to settle in the plate by centrifugation at 200 ×g for 2 minutes at room temperature. By allowing the cells to stand in a $CO_2$ incubator (37°C) for about 2 to 120 minutes, immune cells were brought into contact with the bottom surface of the plate, followed by addition of a fixing solution containing 4% PFA to fix the cell membrane of the immune cells. Next, the same hemolytic agent as in Example 1 was added to perform hemolysis treatment. The cells and cell nuclei were then stained with the same PE-labeled goat anti-mouse IgG antibody, APC-labeled mouse anti-human CD3 antibody and Hoechst as in Example 1. Stained cells were measured by IFC in the same manner as in Example 1. The T cell clone cells seeded on the 48-well plate that had not been immunostimulated prepared in the same manner as above except that the mouse anti-human CD28 antibody had not been added were measured by IFC.

3. Data analysis

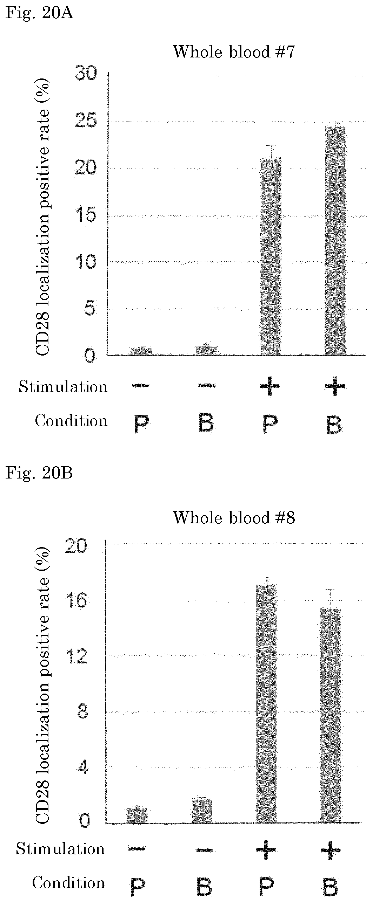

Multiple parameter analysis of the data obtained by IFC measurement was performed in the same manner as in Example 1. The ratio of the T cell clone on which CD28 localized to the T cell clone added to whole blood or the plate (CD28 localization positive rate) was calculated. The results are shown in Figs. 20A and 20B. In the figures, "P" represents the conditions of a conventional method using a plate, and "B" represents the conditions of the method of the invention using whole blood.

4. Result and discussion

As can be seen from Figs. 20A and 20B, no prominent difference in CD28 localization positive rate of T cell clone was observed between the case where the T cell clone was added to whole blood and the case where the T cell clone was seeded in the plate. Until now, blood cell components other than immune cells contained in whole blood have been believed to affect the localization and measurement of target molecules by immunostimulation. However, even when immunostimulation is performed in whole blood, it has been indicated that the method of the invention can detect immune cells exhibiting immunostimulus responsiveness (immune cells exhibiting IS-forming ability), as with a conventional method.

Example 5: Examination of hemolytic agent

Using two types of hemolytic agents, measurement was performed by the method of the invention and a conventional method to compare the CD28 localization positive rate of immune cells. The T cell clone in Example 4 was used as immune cells to be analyzed.

1. Measurement

(1) Measurement of T cell clone by method of the invention

The T cell clone added to whole blood was measured by IFC in the same manner as in Example 4, except for the use of a hemolytic agent 1 (0.8% ammonium chloride) or a hemolytic agent 2 (0.41% ammonium chloride and 0.2% surfactant). Both hemolytic agents 1 and 2 were commercially available products as hemolytic reagents.

(2) Measurement of T cell clone by conventional method

The T cell clone seeded on a 48-well plate was measured by IFC in the same manner as in Example 4.

19

2. Data analysis

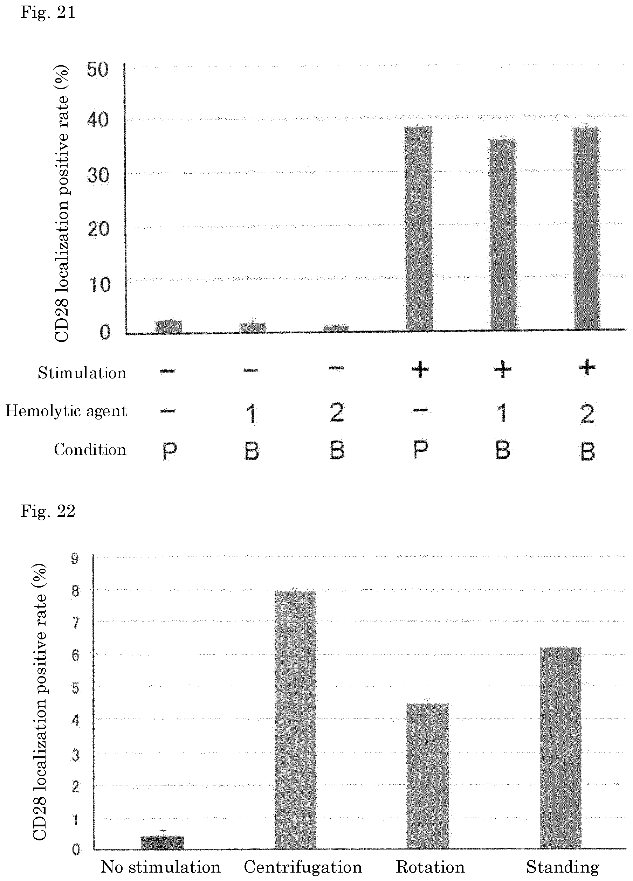

Multiple parameter analysis of the data obtained by IFC measurement was performed in the same manner as in Example 1. The ratio of the T cell clone on which CD28 localized to the T cell clone added to whole blood or the plate (CD28 localization positive rate) was calculated. The results are shown in Fig. 21. In the figures, "P" represents the conditions of a conventional method using a plate, and "B" represents the conditions of the method of the invention using whole blood. In addition, "1" represents that the hemolytic agent 1 is used, and "2" represents that the hemolytic agent 2 is used.

3. Result and discussion

As can be seen from Fig. 21, no prominent difference in CD28 localization positive rate of T cell clone was observed when either hemolytic agent 1 or 2 was used. It has been indicated that the method of the invention using a commercially available hemolytic reagent can detect immune cells exhibiting immunostimulus responsiveness (immune cells exhibiting IS-forming ability).

Example 6: Examination of contact method of immune cells with substance different from immune cells

In the above examples, immunostimulated immune cells were in contact with an erythrocyte layer formed by centrifugation of whole blood. Here, it was examined whether the method of the invention could be carried out based on other contact methods. The T cell clone in Example 4 was used as immune cells to be analyzed.

1. Contact of immune cells with substances different from immune cells

(1) Contact by centrifugation

The Hoechst-labeled T cell clone ($5 \times 10^5$ cells/100 $\mu$L) was added to whole blood (200 $\mu$L) collected from healthy volunteers, and the same anti-human CD28 antibody as in Example 1 was added. A buffy coat was formed by centrifugation in the same manner as in Example 1. By allowing the buffy coat to stand in a $CO_2$ incubator (37°C) for about 2 to 120 minutes, immune cells containing the T cell clone were brought into contact with other leukocytes. In addition, immune cells containing an unstimulated T cell clone were brought into contact with the erythrocyte layer in the same manner as above, except that the anti-human CD28 antibody was not added.

(2) Contact by rotation

The Hoechst-labeled T cell clone ($5 \times 10^5$ cells/100 $\mu$L) was added to whole blood (200 $\mu$L) collected from healthy volunteers, and the same anti-human CD28 antibody as in Example 1 was added. A tube containing whole blood containing a stimulated T cell clone was placed in a rotator and inversion mixed in a $CO_2$ incubator (37°C) at 30 rpm for about 2 to 120 minutes.

(3) Contact by standing

The Hoechst-labeled T cell clone ($5 \times 10^5$ cells/100 $\mu$L) was added to whole blood (200 $\mu$L) collected from healthy volunteers, and the same anti-human CD28 antibody as in Example 1 was added. A tube containing whole blood containing a stimulated T cell clone was allowed to stand in a $CO_2$ incubator (37°C) for about 2 to 120 minutes.

2. Measurement

After providing the contact surface, a fixing solution containing 4% PFA was added to whole blood containing a T cell clone to fix the cell membrane of immune cells. Next, the same hemolytic agent as in Example 1 was added to perform hemolysis treatment. The cells and cell nuclei were then stained with the same PE-labeled goat anti-mouse IgG antibody, APC-labeled mouse anti-human CD3 antibody and Hoechst as in Example 1. Stained cells were measured by IFC in the same manner as in Example 1.

2. Data analysis

Multiple parameter analysis of the data obtained by IFC measurement was performed in the same manner as in Example 1. The ratio of the T cell clone on which CD28 localized to the T cell clone added to whole blood or the plate (CD28 localization positive rate) was calculated. The results are shown in Fig. 22.

3. Result and discussion

As can be seen from Fig. 22, the CD28 localization positive rate showed prominently higher value than that of an unstimulated T cell clone by any contact method of centrifugation, rotation and standing. The CD28 localization positive rate also increased by rotation and standing, suggesting that contact with the inner wall surface of the tube containing whole blood also contributes to immunostimulation of immune cells. It has been indicated that the method of the invention can detect immune cells exhibiting immunostimulus responsiveness (immune cells exhibiting IS-forming ability) by any contact method.

Example 7: Measurement of immunostimulus responsiveness of immune cells in whole blood

By adding allogeneic cells (human B cells) having an immunostimulatory factor to whole blood (peripheral blood) collected from a subject, immune cells (T cells and NK cells) in the whole blood were stimulated, whereby information on the localization of F-actin, a structural protein of the immune cells, was acquired. As a control, measurement was performed for immune cells in whole blood to which no allogeneic cells were added.

1. Measurement

(1) Measurement of immunostimulated immune cells

Blood of a healthy human volunteer (200 μL) was collected in a tube, and to this were added human B cells (RIKEN, product number HEV0032) as allogeneic cells. The resultant whole blood sample was centrifuged to form a buffy coat. After the reaction for 10 minutes or more, the cell membrane of immune cells in the sample was fixed with a fixing solution containing 4% PFA. Next, a hemolytic agent was added to the fixed cells to perform hemolysis treatment. The cells were then stained with Alexa488-mouse anti-human CD3 antibody, PE-Cy7-mouse anti-human CD56 antibody, and Alexa674-phalloidin. Stained cells were measured using IFC (ImageStreamX Mark II Imaging Flow Cytometer: Amnis).

(2) Measurement of non-immunostimulated immune cells

Blood of a healthy human volunteer (200 μL) was collected in a tube and centrifuged to form a buffy coat. After the reaction for 2 minutes or more, the cell membrane of immune cells in the sample was fixed with a fixing solution containing 4% PFA. Next, a hemolytic agent was added to the fixed cells to perform hemolysis treatment. The cells were then stained with Alexa488-mouse anti-human CD3 antibody, PE-Cy7-mouse anti-human CD56 antibody, and Alexa674-phalloidin. Stained cells were measured using IFC (ImageStreamX Mark II Imaging Flow Cytometer: Amnis).

2. Data analysis

Multi-parameter analysis was performed on the measurement data on the whole blood containing stimulated immune cells and the measurement data on the whole blood containing unstimulated immune cells obtained by measurement by IFC. Briefly, image analysis was performed based on transmitted light images and fluorescence images (CD3, CD56 and F-actin) of individual cells obtained by IFC, whereby cell size, cell aspect ratio, fluorescence signal area value, and total fluorescence signal intensity were acquired. First, based on the transmitted light image data on individual cells, a 2D scattergram was created in which the cell size was assigned on the X-axis and the cell aspect ratio was assigned on the cells on the Y-axis (not shown). In order to exclude the measurement data on debris such as erythrocyte ghosts, dots within a predetermined range (Cells) of the 2D scattergram were selected, and the measurement data corresponding to each selected dot were extracted as the measurement data on a cell.

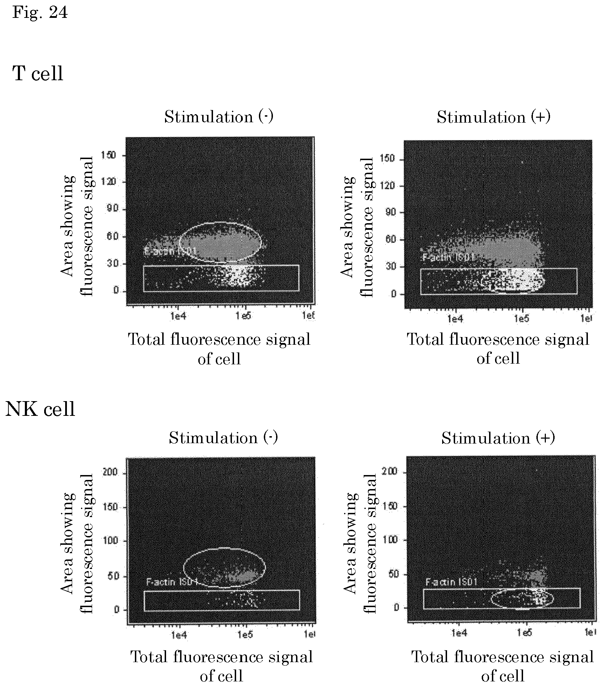

Then, based on the measurement data within the range (Cells), a 2D scattergram was created in which the total fluorescence signal intensity acquired from the fluorescence image (CD3) was assigned on the X-axis, and the total fluorescence signal intensity acquired from the fluorescence image (CD56) was assigned on the Y-axis (see Fig. 24). In the 2D scattergram, a dot whose CD3 total fluorescence signal intensity was higher than or equal to a predetermined value and whose CD56 total fluorescence signal intensity was lower than or equal to a predetermined value was defined as a CD3-positive and CD56-negative immune cell (hereinafter, the immune cell is also referred to as "CD3$^+$CD56$^-$ cell"). In addition, in the 2D scattergram, a dot whose CD3 total fluorescence signal intensity was equal to or lower than a predetermined value and whose CD56 total fluorescence signal intensity was higher than or equal to a predetermined value was defined as a CD3-negative and CD56-positive immune cell (hereinafter, the immune cell is also referred to as "CD3⁻CD56⁺ cell").

Based on the data acquired from the fluorescence image (F-actin) of CD3⁺CD56⁻cells, a 2D scattergram was created in which the total fluorescence signal intensity was assigned on the X-axis and the fluorescence signal area value was assigned on the Y-axis (not shown). In the 2D scattergram, dots within the range (T cells) where the total fluorescence signal intensity was higher than or equal to a predetermined value were selected. The measurement data corresponding to each selected dot were extracted as the measurement data on an immune cell (T cell). For the extracted measurement data, a 2D scattergram was created in which the total fluorescence signal intensity was assigned on the X-axis and the fluorescence signal area value was assigned on the Y-axis. Then, from the measurement data within the range (T cells), the measurement data on a cell population having a low fluorescence signal area value were extracted. Specifically, in the created 2D scattergram, dots within a predetermined range (F-actin IS) were selected, and measurement data corresponding to each selected dot were extracted as the measurement data on an immunostimulus responsive T cell. IS is an abbreviation for immunological synapse.

Similarly, based on the data on the fluorescence image (F-actin) of CD3⁻CD56⁺ cells, measurement data on immunostimulus responsive NK cells were extracted. Based on the data acquired from the fluorescence image (F-actin) of CD3⁻CD56⁺ cells, a 2D scattergram was created in which the total fluorescence signal intensity was assigned on the X-axis and the fluorescence signal area value was assigned on the Y-axis (not shown). In the 2D scattergram, dots within the range (NK cells) where the total fluorescence signal intensity was higher than or equal to a predetermined value were selected. The measurement data corresponding to each selected dot were extracted as the measurement data on an immune cell (NK cell). For the extracted measurement data, a 2D scattergram was created in which the total fluorescence signal intensity was assigned on the X-axis and the fluorescence signal area value was assigned on the Y-axis. Then, from the measurement data within the range (NK cells), the measurement data on a cell population having a low fluorescence signal area value were extracted. Specifically, in the created 2D scattergram, dots within a predetermined range (F-actin IS) were selected, and measurement data corresponding to each selected dot were extracted as the measurement data on an immunostimulus responsive NK cell.

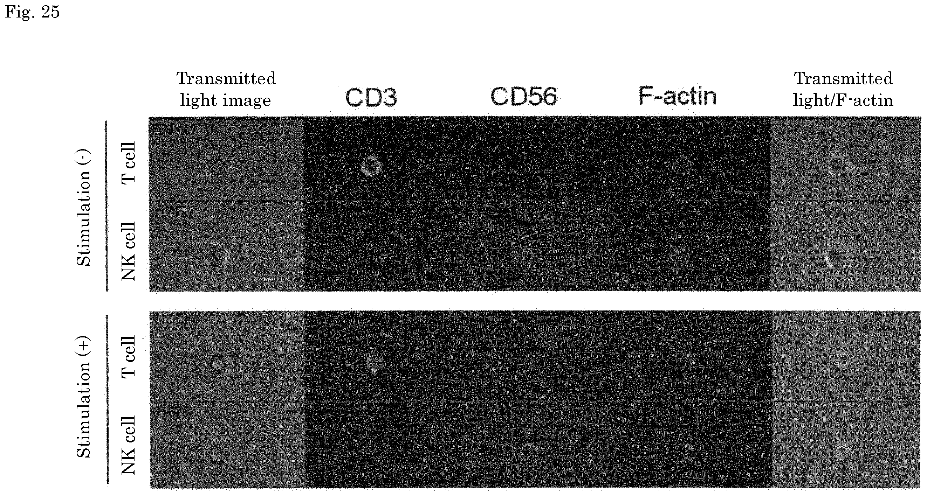

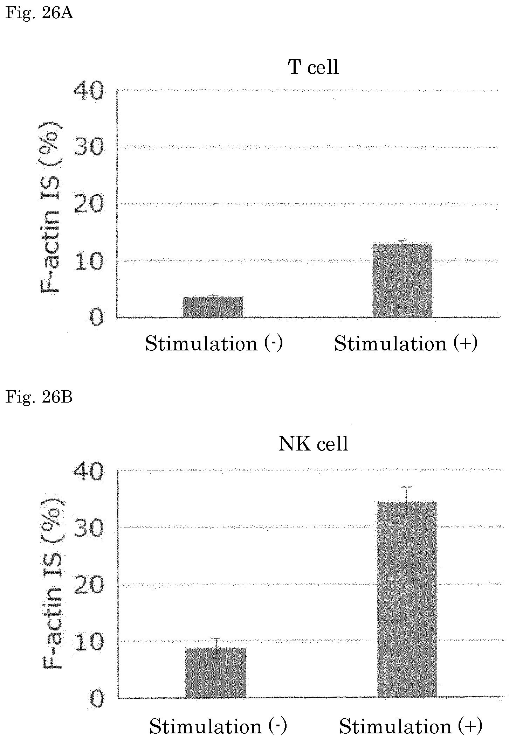

Fig. 24 shows an example of a 2D scattergram created based on the measurement data in the range (T cells) or range (NK cells). In the figure, stimulation (-) represents the distribution of immune cells that have not been immunostimulated, and stimulation (+) represents the distribution of immune cells that have been immunostimulated. In the figures, the region surrounded by a rectangle is the range (F-actin IS). In addition, an example of the image of a cell corresponding to each dot within the region surrounded by an ellipse in Figs. 24 is shown in Figs. 25. Dots within the range (T cells) and range (F-actin IS01) were counted, and the ratio of T cells on which F-actin localized to T cells in whole blood (F-actin localization positive rate) was calculated from the following formula. Similarly, dots within the range (NK cells) and range (F-actin IS01) were counted, and the ratio of NK cells on which F-actin localized to NK cells in whole blood (F-actin localization positive rate) was calculated from the following formula. The F-actin localization positive rates of T cells and NK cells are shown in Figs. 26A and 26B.

$$[\text{F-actin localization positive rate (\%)}] = \{[\text{number of dots within range (F-actin IS)}]/[\text{number of dots within range (T cells)}]\} \times 100$$

$$[\text{F-actin localization positive rate (\%)}] = \{[\text{number of dots within range (F-actin IS)}]/[\text{number of dots within range (NK cells)}]\} \times 100$$

3. Result and discussion

On T cells and NK cells that had not been brought into contact with an immunostimulatory factor, including a human B cell line, F-actin molecules were uniformly distributed on the cell membrane. On the other hand, when immunostimulated, it has been found that the F-actin molecules localize on a part of the membrane of T cells and NK cells. It is believed that the portion showing this localization represents a formed immunological synapse (see Fig. 25). In the 2D scattergram are shown information reflecting the area value of the F-actin fluorescence signal on cells on the Y-axis and information reflecting the total fluorescence signal value of F-actin on cells on the X-axis (see Fig. 24). Here, since cells whose localized area shows a small value tend to have IS formed, this region is gated, whereby a region where immune cells exhibiting immunostimulus responsiveness appear is shown. With reference to Fig. 24, comparing the 2D scattergrams with and without stimulation, it can be seen that cells are prominently appearing in the gating area in the case with stimulation. From Figs. 26A and 26B, the IS positive rates of T cells and NK cells in whole blood showed very low values without stimulation. Compared to the case without stimulation, the value of IS positive rate of T cells and NK cells in whole blood significantly increased with stimulation.

From the above, it has been indicated that the method of the Examples using whole blood can detect immune cells exhibiting immunostimulus responsiveness (immune cells exhibiting immune cells exhibiting IS-forming ability), and can evaluate immunostimulus responsiveness (IS-forming ability) of immune cells based on the detection results.

DESCRIPTION OF REFERENCE SIGNS

1: Cell analysis instrument
2: Measuring device
3: Information processing device
300: Main body
310: Input unit
320: Display unit

**Claims**

1.  A method for analyzing immune cells, comprising steps of

    mixing whole blood containing immune cells with an immunostimulatory factor *in vitro* and labeling target molecules of the immune cells, and
    acquiring information on localization of the target molecules on the immune cells by detecting the label.

2.  The method according to claim 1, wherein the step of labeling target molecules comprises contacting the immune cells with a substance different from the immune cells.

3.  The method according to claim 2, wherein the contacting step is performed by centrifuging, stirring, or allowing to stand a mixture of the whole blood containing immune cells with the immunostimulatory factor.

4.  The method according to claim 2 or 3, wherein the substance different from immune cells is blood cells other than the immune cells having target molecules labeled, or an inner surface of a container accommodating the whole blood.

5.  The method according to any one of claims 2 to 4, wherein the contacting step is performed by centrifuging a mixture of the whole blood containing immune cells with the immunostimulatory factor to form a layer containing the immune cells and a layer containing erythrocytes.

6.  The method according to any one of claims 1 to 5, comprising a step of fixing the immune cells between the step of labeling target molecules and the step of acquiring information on localization of target molecule.

7.  The method according to any one of claims 1 to 6, comprising a step of lysing erythrocytes contained in the whole blood between the step of labeling target molecules and the step of acquiring information on localization of target molecule.

8.  The method according to claim 7 referring to claim 6, wherein the step of lysing erythrocytes is performed between the step of fixing immune cells and the step of acquiring information on localization of target molecule.

9.  The method according to any one of claims 1 to 8, wherein, in the step of acquiring information on localization of target molecule, the target molecules are labeled by binding the target molecules of the immune cells, a trapping body that binds to the target molecules, and a labeling substance that can generate a detectable optical signal.

10. The method according to claim 9, wherein the immunostimulatory factor is shared as the trapping body that binds to target molecules.

11. The method according to claim 9 or 10, wherein the labeling substance labels the target molecules by directly or indirectly binding to the trapping body.

**12.** The method according to any one of claims 9 to 11, comprising detecting an optical signal generated from the labeling substance and acquiring the information on localization of target molecule on the immune cells based on the detected optical signal.

**13.** The method according to claim 12, comprising, based on the optical signal, acquiring a value reflecting a distribution of the labeled target molecules on immune cell, and based on the acquired value, counting the immune cells on which target molecules localize.

**14.** The method according to any one of claims 1 to 13, wherein the immune cells contained in whole blood is a T cell subset or an NK cell subset.

**15.** The method according to claim 14, comprising, based on a counted number of the immune cells on which target molecules localize, acquiring an index of immunostimulus responsiveness of the T cell subset or the NK cell subset.

**16.** The method according to claim 15, comprising counting a total number of the immune cells contained in whole blood, and based on the counted number of the immune cells on which target molecules localize and the total number, acquiring an index of immunostimulus responsiveness of the T cell subset or the NK cell subset.

**17.** The method according to any one of claims 1 to 16, wherein the target molecules is at least one selected from a surface antigen and a polymer of structural protein.

**18.** The method according to claim 17, wherein the surface antigen is molecules constituting an immunological synapse.

**19.** The method according to claim 17 or 18, wherein the surface antigen is at least one selected from CD28, CD3, TCR$\alpha$/$\beta$, CD40L, OX40, CTLA4, PD-1 and ICOS, and the polymer of structural protein is F-actin.

**20.** The method according to any one of claims 1 to 19, wherein the immunostimulatory factor comprises at least one selected from the group consisting of an immunostimulatory antibody, an immunostimulatory peptide, a major histocompatibility molecule (MHC molecule), and a complex of an MHC molecule with an antigenic peptide.

**21.** The method according to any one of claims 1 to 19, wherein the immunostimulatory factor comprises an immunostimulatory factor present on at least one cell selected from the group consisting of an allogeneic cell, an antigen-presenting cell and a tumor cell.

**22.** The method according to any one of claims 1 to 21, wherein a stimulation time of the immune cells with the immunostimulatory factor is less than 6 hours.

**23.** A method for analyzing an immune cell, comprising steps of

acquiring information on localization of a target molecule on an immune cell by detecting the label of the immune cell having a labeled target molecule,
wherein the immune cell containing the labeled target molecule is a cell prepared by mixing whole blood containing immune cells with an immunostimulatory factor *in vitro* and labeling target molecules of the immune cells contained in whole blood.

**24.** A cell analysis instrument, comprising

an introducing unit that introduces a complex of an immune cell, a trapping body that binds to a target molecule of the immune cell, and a labeling substance that can generate a detectable optical signal,
an image capturing unit that captures the complex supplied from the introducing unit, and
an analyzing unit that acquires information on localization of the target molecule on the immune cell based on an image captured by the image capturing unit,
wherein the immune cell is a cell obtained by mixing whole blood containing the immune cells with an immunostimulatory factor *in vitro* to localize the target molecules on the immune cells.

**25.** A cell analysis instrument, comprising

an introducing unit that introduces a complex of an immune cell, a trapping body that binds to a target molecule of the immune cell, and a labeling substance that can generate a detectable optical signal,

a detection unit that irradiates the complex supplied from the introducing unit with light to detect the optical signal generated from the complex, and

an analyzing unit that acquires information on localization of the target molecule on the immune cell based on the detected optical signal,

wherein the immune cell is a cell obtained by mixing whole blood containing the immune cells with an immunostimulatory factor *in vitro* to localize the target molecules on the immune cells.

Fig. 1

| | Immunostimulatory factor and trapping body are shared | Immunostimulatory factor and trapping body are separated |
|---|---|---|
| Direct bond of trapping body to labeling substance | A<br>Immunostimulatory factor and trapping body / Labeling substance / Target molecule / Immune cell | C<br>Immunostimulatory factor / Labeling substance / Trapping body / Target molecule |
| Indirect bond of trapping body to labeling substance | B<br>Immunostimulatory factor and trapping body / Labeling substance / Substance that specifically binds to trapping body / Target molecule | D<br>Immunostimulatory factor / Trapping body / Labeling substance / Substance that specifically binds to trapping body / Target molecule |

EP 3 889 601 A1

Fig. 2

Fig. 3

Fig. 4

Fig. 5

203ib

203ic

203ia

Fig. 6

Recording medium

300

301 CPU

302 ROM

303 RAM

304 Hard disk

305 Read-out device

306 Input/output interface

307 Image output interface

308 Communication interface

310 Input unit

320 Display unit

3

2 Measuring device

Fig. 7

(a)

```
        Start

         ↓
    ┌─────────────┐
NO  │   Receive   │  S 2 1
←───│ measurement │
    │   start     │
    │   signal?   │
    └─────────────┘
         ↓ YES

S 2 2
    ┌─────────────┐
    │   Measure   │
    └─────────────┘
         ↓

S 2 3
    ┌─────────────┐
    │  Transmit   │
    │ measurement │
    │    data     │
    └─────────────┘
         ↓
        End
```

(b)

```
        Start

         ↓
S 1 1
    ┌─────────────┐
    │ Measurement │  NO
    │   start     │───→
    │ instruction?│
    └─────────────┘
         ↓ YES

    ┌─────────────┐  S 1 2
    │  Transmit   │
    │ measurement │
    │ start signal│
    └─────────────┘
         ↓

S 1 3
    ┌─────────────┐
    │   Receive   │  NO
    │ measurement │───→
    │    data?    │
    └─────────────┘
         ↓ YES

    ┌─────────────┐  S 1 4
    │ Measurement │
    │  processing │
    └─────────────┘
         ↓

    ┌─────────────┐  S 1 5
    │  Analysis   │
    │  processing │
    └─────────────┘
         ↓

    ┌─────────────┐  S 1 6
    │Display analysis│
    │   result    │
    └─────────────┘
         ↓
        End
```

31

Fig. 8

|  | Transmitted light image | Fluorescence image | Merge |
|---|---|---|---|

(a)

(b)

Fig. 9

| Transmitted light image | Fluorescence image |
|---|---|

| | |
|---|---|
| Cell size | |
| Aspect ratio | |
| Area showing fluorescence signal (fluorescence signal area value) | |
| Total fluorescence signal intensity within above-mentioned area | |

Fig. 10

```
          ┌─────────────────┐
          │   Analysis      │
          │   processing    │
          └────────┬────────┘
                   │
                   ▼
S51  ┌────────────────────────────────┐
     │  Extract measurement data on   │
     │  cell whose aspect ratio and   │
     │  size are within predetermined │
     │  range (R1)                    │
     └────────────────┬───────────────┘
                      │
                      ▼
S52  ┌────────────────────────────────┐
     │  Extract measurement data on   │
     │  cell whose total fluorescence │
     │  signal intensity and sharpness│
     │  are within predetermined      │
     │  range (R2)                    │
     └────────────────┬───────────────┘
                      │
                      ▼
S53  ┌────────────────────────────────┐
     │  Extract measurement data on   │
     │  cell whose fluorescence signal│
     │  area value and total          │
     │  fluorescence signal intensity │
     │  are within predetermined      │
     │  range (R3)                    │
     └────────────────┬───────────────┘
                      │
                      ▼
S54  ┌────────────────────────────────┐
     │  Calculate ratio of number of  │
     │  dots within predetermined     │
     │  range (R3) to number of dots  │
     │  within predetermined          │
     │  range (R2)                    │
     └────────────────┬───────────────┘
                      │
                      ▼
              ┌──────────────┐
              │   Return     │
              └──────────────┘
```

Fig. 11

Fig. 12

Fig. 13

(a)

Cell

(b)

(c)

Fluorescence intensity

Baseline

Area (A)

Pulse width (W)

Pulse height (H)

Time

(d)

Fluorescence intensity

Time

Fig. 14

a

Pulse width (W)

Pulse height (H)

b

Pulse area (A)

Pulse height (H)

Fig. 15A

Fig. 15B

Fig. 16A

Fig. 16B

Fig. 17

Fig. 18

Fig. 19

Fig. 20A

Whole blood #7

Fig. 20B

Whole blood #8

Fig. 21

Fig. 22

Fig. 23

CD3-negative and CD56-positive immune cell

CD3-positive and
CD56-negative immune cell

Total fluorescence signal intensity (CD56)

Total fluorescence signal intensity (CD3)

Fig. 24

## T cell

Stimulation (·)

Stimulation (+)

Total fluorescence signal of cell

Total fluorescence signal of cell

## NK cell

Stimulation (·)

Stimulation (+)

Total fluorescence signal of cell

Total fluorescence signal of cell

Fig. 25

Fig. 26A

T cell

Fig. 26B

NK cell

## INTERNATIONAL SEARCH REPORT

International application No.

PCT/JP2019/046410

### A. CLASSIFICATION OF SUBJECT MATTER

Int.Cl. G01N33/49(2006.01)i, G01N33/53(2006.01)i
FI: G01N33/49A, G01N33/53K

According to International Patent Classification (IPC) or to both national classification and IPC

### B. FIELDS SEARCHED

Minimum documentation searched (classification system followed by classification symbols)
Int.Cl. G01N33/48-33/98

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2018-179709 A (SYSMEX CORPORATION) 15.11.2018 (2018-11-15), paragraphs [0036]-[0039], [0042]-[0046], [0081]-[0128], [0134]-[0167], fig. 1-25 | 1-6, 9-25<br>7-8 |
| Y | JP 2016-186463 A (SYSMEX CORPORATION) 27.10.2016 (2016-10-27), paragraphs [0009]-[0031], fig. 1, 2 | 7-8 |
| Y | JP 2014-228285 A (HORIBA LTD.) 08.12.2014 (2014-12-08), paragraphs [0017]-[0022], fig. 1, 2 | 7-8 |
| A | JP 2013-183664 A (UNIVERSITY OF TOYAMA) 19.09.2013 (2013-09-19), paragraphs [0070]-[0084] | 1-25 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14.02.2020 | 03.03.2020 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2019/046410

C (Continuation). DOCUMENTS CONSIDERED TO BE RELEVANT

| Category* | Citation of document, with indication, where appropriate, of the relevant passages | Relevant to claim No. |
|---|---|---|
| A | TSENG, S. Y. et al., CD80 cytoplasmic domain controls localization of CD28, CTLA-4, and protein kinase Cθ in the immunological synapse, The Journal of Immunology, 15 December 2005, vol. 175, no. 12, pp. 7829-7836, entire text | 1-25 |
| A | WO 2018/092754 A1 (ONCOTHERAPY SCIENCE, INC.) 24.05.2018 (2018-05-24), claim 27 | 1-25 |
| A | US 2014/0186372 A1 (ALTMAN, A.) 03.07.2014 (2014-07-03), paragraphs [0003]-[0023] | 1-25 |

Form PCT/ISA/210 (second sheet) (January 2015)

## INTERNATIONAL SEARCH REPORT
Information on patent family members

International application No.

PCT/JP2019/046410

| | | |
|---|---|---|
| JP 2018-179709 A | 15.11.2018 | US 2018/0292385 A1<br>paragraphs [0060]-[0063], [0066]-[0070],<br>[0105]-[0152],<br>[0156]-[0182], fig. 1-25<br>EP 3388832 A1<br>CN 108761054 A |
| JP 2016-186463 A | 27.10.2016 | US 2016/0282346 A1<br>paragraphs [0028]-[0050], fig. 1, 2<br>EP 3073265 A1<br>CN 106018771 A |
| JP 2014-228285 A | 08.12.2014 | US 2014/0341780 A1<br>paragraphs [0061]-[0086], fig. 1, 2<br>EP 2804003 A1<br>CN 104165834 A |
| JP 2013-183664 A | 19.09.2013 | US 2015/0038363 A1<br>paragraphs [0102]-[0116]<br>WO 2013/133297 A1<br>EP 2824182 A1 |
| WO 2018/092754 A1 | 24.05.2018 | JP 2019-535270 A<br>claim 27 |
| US 2014/0186372 A1 | 03.07.2014 | WO 2012/174412 A2<br>paragraphs [0003]-[0023]<br>EP 2720705 A2 |

Form PCT/ISA/210 (patent family annex) (January 2015)

**EP 3 889 601 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180292385 A